# EUROPEAN PATENT APPLICATION

(11) **EP 3 593 816 A1**
(43) Date of publication of application: **15.01.2020**
(21) Application number: 18763814.3
(22) Date of filing: 06.03.2018
(51) Int. Cl.: A61K 45/00, A61K 31/713, A61K 38/18, A61K 38/48, A61K 39/395, A61K 48/00, A61P 9/00

(54) **LYPD1 INHIBITOR AND METHOD FOR PRODUCING BIOLOGICAL TISSUE USING SAME**

(30) Priority: 06.03.2017 JP 2017042200
(71) Applicant: Tokyo Women's Medical University, Tokyo 162-8666 (JP)
(72) Inventor: MATSUURA, Katsuhisa, Tokyo 162-8666 (JP); SHIMIZU, Tatsuya, Tokyo 162-8666 (JP); AOKI, Shinako, Tokyo 162-8666 (JP); SAKAMOTO, Satoru, Tokyo 162-8666 (JP)
(74) Representative: Ricker, Mathias
(86) International application number: PCT/JP2018/008630
(87) International publication number: WO 2018/164141

(57) **Abstract**

Provided is a LYPD1 inhibitor for promoting vascular endothelial network formation in a biological tissue. Also provided is a medicinal composition, said medicinal composition comprising a LYPD1 inhibitor as an active ingredient, for treating and/or preventing neoangiogenic disorders. Also provided is a method which comprises: (a1) a step for providing a cell population containing first cells expressing LYPD1 and vascular endothelial cells; (a2) a step for treating the cell population obtained in step (a1) with a LYPD1 inhibitor; and (a3) a step for culturing the cell population obtained in step (a2).

## Description

### FIELD

The present invention relates to a LYPD1 inhibitor for promoting vascular endothelial network formation in biological tissue. In addition, the present invention relates to a method for producing biological tissue in which vascular endothelial network formation has been promoted. Furthermore, the present application claims priority on the basis of Japanese Patent Application No. 2017-42200 filed at the Japan Patent Office on March 6, 2017 and the entire description thereof is incorporated in the present description by reference.

### BACKGROUND

Ischemic heart disease is the second leading cause of death in Japan and is currently considered to be one of the most important diseases requiring a solution. A wide range of treatment methods for promoting angiogenesis, such as administration of an angiogenesis induction factor such as vascular endothelial growth factor (VEGF) or transplantation of vascular endothelial progenitor cells, have previously been developed as angiogenic therapy methods for ischemic heart disease. On the other hand, these treatment methods have the risk of promoting angiogenesis throughout the body, making it difficult to apply these methods to cancer patients. In addition, in the case of using an angiogenic growth factor, adverse side effects such as vascular edema end up occurring, thereby resulting in obstacles to application in the clinical setting.

Treatment methods using a cell sheet have been developed as treatment methods that only promote angiogenesis of the organ or tissue targeted for treatment without promoting angiogenesis in organs or tissues throughout the body. For example, treatment methods using a cell sheet have been developed as treatment methods for treating heart diseases including ischemic heart disease, and a portion thereof are used clinically (refer to PTL1 to PTL3).

However, the cell sheets described in PTL1 to PTL3 comprise treatment methods that demonstrate therapeutic effects through the action of substances such as cytokines secreted from the cell sheet into the affected area where the treatment method is applied, and are considered to be inadequate treatment methods for the treatment of organs or tissues that have undergone irreversible damage due to a serious disease. Consequently, in order to treat such organs or tissues, it is thought to be necessary to replace (transplant) with biological tissue having a function that takes the place of that site.

Various tissue engineering techniques have been developed as techniques for fabricating biological tissue capable of taking the place of diseased organs or tissues. In particular, attempts have been made to develop a method for constructing biological tissue having a certain degree of thickness. In order to construct biological tissue having a certain degree of thickness, it is necessary to construct a vascular endothelial network within the biological tissue to allow the construction of a functional vascular network. For example, the methods described in PLT4 and PTL5 are disclosed as methods for constructing biological tissue having a functional vascular network and a certain degree of thickness in vitro.

The aforementioned methods are not adequate for obtaining biological tissue in which a functional vascular network has been constructed therein, thus resulting in the need for the development of a novel method for easily constructing a functional vascular network.

### [CITATION LIST]

### [PATENT LITERATURE]

[PTL1] International Publication No. WO 2005/011524
[PTL2] International Publication No. WO 2011/067983
[PTL3] International Publication No. WO 2014/148321
[PTL4] International Publication No. WO 2012/036224
[PTL5] International Publication No. WO 2012/036225

### SUMMARY

### [TECHNICAL PROBLEM]

An object of the present invention is to solve the aforementioned problems in order to easily promote the formation of a vascular endothelial network in biological tissue.

### [SOLUTION TO PROBLEM]

The inventors of the present invention conducted extensive studies in addition to research and development from various perspectives in order to solve the aforementioned problems. As a result, the inventors of the present invention surprisingly found that the formation of a vascular endothelial network is promoted in biological tissue by inhibiting LYPD1. Namely, the present invention provides the inventions indicated below.
[1] An LYPD1 inhibitor for promoting vascular endothelial network formation in biological tissue.
[2] The LYPD1 inhibitor described in [1] for treating and/or preventing angiogenic disorders.
[3] The LYPD1 inhibitor described in [2], wherein the angiogenic disorder is selected from the group consisting of cerebrovascular disease, cerebral infarction, transient ischemic attack, moyamoya disease, angina, (peripheral) arterial occlusion, arteriosclerosis, Buerger's disease, myocardial infarction, ischemia, cardiomyopathy, congestive heart failure, coronary artery disease, hereditary hemorrhagic telangiectasia, ischemic heart disease, vascular intimal thickening, vascular occlusion, atherosclerotic peripheral vascular disease, portal hypertension, rheumatic heart disease, hypertension, thromboembolism, atherosclerosis, post-angioplasty restenosis, pulmonary arterial hypertension, vein graft disease, hypertensive heart disease, valvular heart disease, Kawasaki disease, dilated cardiomyopathy, hypertrophic cardiomyopathy, sarcoidosis, systemic scleroderma, aortitis syndrome, asymptomatic myocardial ischemia, internal carotid artery stenosis, vertebral artery stenosis, hemodialysis cardiomyopathy, diabetic cardiomyopathy, pulmonary arterial pulmonary hypertension, ischemic cardiomyopathy, post-coronary artery bypass surgery, post-percutaneous transluminal coronary angioplasty, acute myocardial infarction, subacute myocardial infarction, old myocardial infarction, exertional angina, unstable angina, acute coronary syndrome, coronary vasospastic angina, aortic valve stenosis, aortic valve insufficiency, mitral valve insufficiency and mitral valve stenosis.
[4] The LYPD1 inhibitor described in [2], wherein the angiogenic disorder is selected from the group consisting of angina, myocardial infarction, cardiomyopathy, congestive heart failure, coronary artery disease, ischemic heart disease, rheumatic heart disease, post-angioplasty restenosis, hypertensive heart disease, valvular heart disease, Kawasaki disease, dilated cardiomyopathy, hypertrophic cardiomyopathy, systemic scleroderma, aortitis syndrome, asymptomatic myocardial ischemia, internal carotid artery stenosis, vertebral artery stenosis, hemodialysis cardiomyopathy, diabetic cardiomyopathy, pulmonary artery pulmonary hypertension, ischemic cardiomyopathy, post-coronary bypass surgery, post-percutaneous transluminal coronary angioplasty, acute myocardial infarction, subacute myocardial infarction, old myocardial infarction, exertional angina, unstable angina, acute coronary syndrome, coronary vasospastic angina, aortic valve stenosis, aortic valve insufficiency, mitral valve insufficiency and mitral valve stenosis.
[5] The LYPD1 inhibitor described in any of [1] to [4], wherein the biological tissue is biological tissue that expresses LYPD1.
[6] The LYPD1 inhibitor described in any of [1] to [5], wherein the LYPD1 inhibitor is a selective LYPD1 inhibitor.
[7] The LYPD1 inhibitor described in [6], wherein the selective LYPD1 inhibitor is selected from the group consisting of an organic small molecule, an aptamer, an antibody, an antibody fragment and a combination thereof.
[8] The LYPD1 inhibitor described in any of [1] to [5], wherein the LYPD1 inhibitor is a LYPD1 expression inhibitor or cells treated with a LYPD1 expression inhibitor.
[9] The LYPD1 inhibitor described in [8], wherein the cells are provided in the form of a cell suspension or cell sheet.
[10] The LYPD1 inhibitor described in [8] or [9], wherein the LYPD1 expression inhibitor is selected from the group consisting of an antisense RNA or DNA molecule, an RNAi-inducing nucleic acid, a microRNA (miRNA), a ribozyme, a genome-editing nucleic acid and expression vector thereof, an organic small molecule, an aptamer, an antibody, an antibody fragment and a combination thereof.
[11] A pharmaceutical composition for treating and/or preventing angiogenic disorders comprising as an active ingredient thereof the LYPD1 inhibitor described in any of [1] to [10].
[12] The pharmaceutical composition described in [11], further comprising one or more angiogenesis induction factors selected from the group consisting of vascular endothelial growth factor (VEGF), hepatocyte growth factor (HGF), fibroblast growth factor (FGF), epidermal growth factor (EGF), platelet-derived growth factor (PDGF), insulin-like growth factor (IGF), angiopoietin, transforming growth factor-β (TGF-β), placental growth factor (PIGF), matrix metalloproteinase (MMP), family proteins thereof, and combinations thereof.
[13] A method for producing biological tissue in which vascular endothelial network formation has been promoted, comprising:
   (a1) a step for providing a cell population containing first cells expressing LYPD1 and vascular endothelial cells and/or vascular endothelial progenitor cells,
   (a2) a step for treating the cell population obtained in step (a1) with an LYPD1 inhibitor, and
   (a3) a step for culturing the cell population obtained in step (a2); or
   (b1) a step for treating a cell population containing first cells expressing LYPD1 with an LYPD1 inhibitor,
   (b2) a step for contacting vascular endothelial cells and/or vascular endothelial progenitor cells with the cell population obtained in step (b1), and
   (b3) a step for culturing the cell population obtained in step (b2).
[14] The method described in [13], wherein the first cells are cells derived from the heart, muscle, kidney and/or brain.
[15] The method described in [13] or [14], wherein the LYPD1 inhibitor is selected from the group consisting of an antisense RNA or DNA molecule, an RNAi-inducing nucleic acid, a microRNA (miRNA), a ribozyme, a genome-editing nucleic acid and expression vector thereof, cells in which the expression vector has been introduced, second cells in which the expression level of LYPD1 is lower than the expression level of LYPD1 of the first cells or is not expressed at all, an organic small molecule, an aptamer, an antibody, an antibody fragment and a combination thereof.
[16] The method described in [15], wherein the second cells are cells derived from the skin, esophagus, lung and/or liver.
[17] A use of the LYPD1 inhibitor described in any of [1] to [10] for producing a pharmaceutical composition for treating and/or preventing angiogenic disorders.
[18] A method for screening LYPD1 inhibitors, comprising:
   (i-1) a step for providing a cell population containing first cells expressing LYPD1 and vascular endothelial cells and/or vascular endothelial progenitor cells,
   (i-2) a step for treating the cell population obtained in step (i-1) with a candidate substance,
   (i-3) a step for culturing the cell population obtained in step (i-2), and
   (i-4) a step for evaluating formation of a vascular endothelial network in the cell population obtained in step (i-3); or,
   (ii-1) a step for treating a cell population containing first cells expressing LYPD1 with a candidate substance,
   (ii-2) a step for contacting vascular endothelial cells and/or vascular endothelial progenitor cells with the cell population obtained in step (ii-1),
   (ii-3) a step for culturing the cell population obtained in step (ii-2), and
   (ii-4) a step for evaluating formation of a vascular endothelial network in the cell population obtained in step (ii-3).
[19] The method described in [18], wherein the first cells are cells derived from the heart, muscle, kidney and/or liver, or cells in which a vector expressing LYPD1 has been introduced.

### [ADVANTAGEOUS EFFECTS OF INVENTION]

According to the present invention, vascular endothelial network formation can be promoted in biological tissue. In particular, according to the present invention, angiogenesis can be promoted in biological tissue highly expressing LYPD1 and having an angiogenic disorder. In addition, according to the present invention, construction of a vascular endothelial network can be promoted in a subject having an angiogenic disorder. Moreover, according to the present invention, biological tissue can be provided in which vascular endothelial network formation has been promoted.

### BRIEF DESCRIPTION OF DRAWINGS

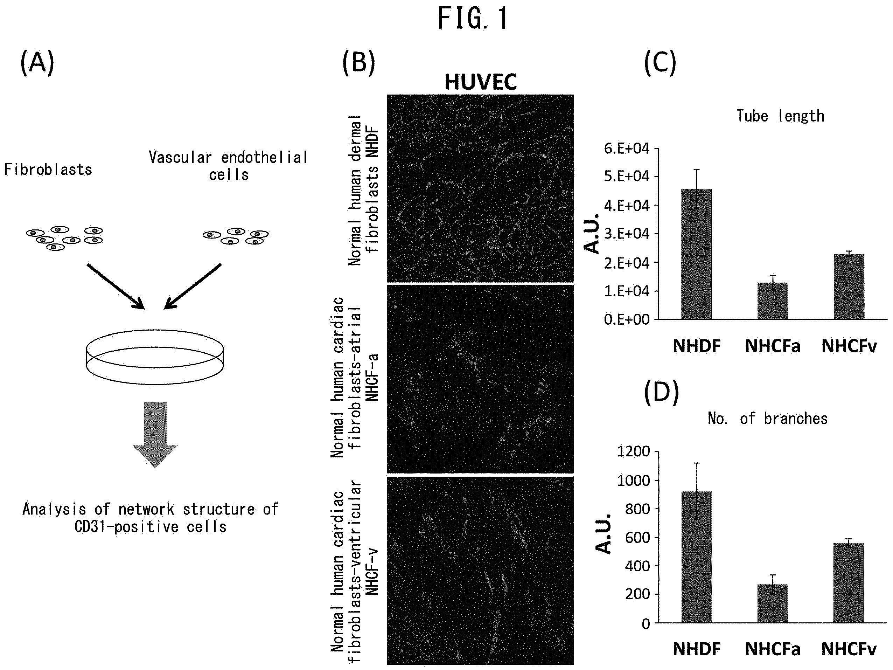

FIG. 1 depicts drawings indicating inhibition of vascular endothelial network formation by cardiac fibroblasts. (A) is a drawing indicating the procedure of the present example. (B) depicts the results of immunostaining with anti-CD31 antibody following co-culturing of normal human dermal fibroblasts (NHDF) or normal human cardiac fibroblasts (atrial fibroblasts represented by NHCF-a and ventricular fibroblasts represented by NHCF-v) with human umbilical vein endothelial cells (HUVEC). The green color indicates CD31-positive cells. (C) depicts a graph indicating the total lengths of the vascular endothelial networks indicated in (B). (D) depicts a graph indicating the number of branches in the vascular endothelial networks indicated in (B).

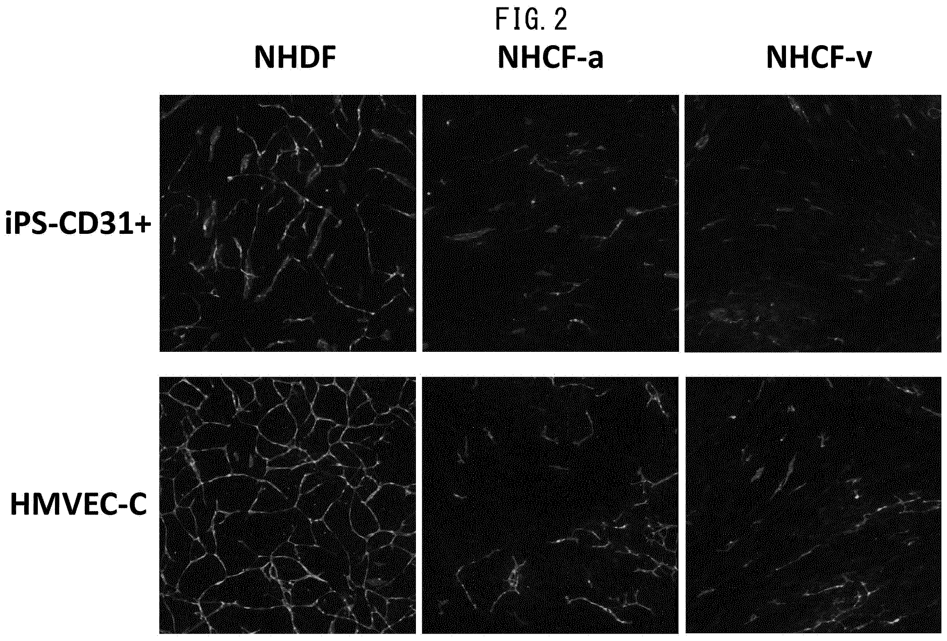

FIG. 2 depicts vascular endothelial networks following co-culturing of normal human dermal fibroblasts (NHDF) or cardiac fibroblasts (atrial fibroblasts represented by NHCF-a and ventricular fibroblasts represented by NHCF-v) with iPS cell-derived vascular endothelial cells (iPS-CD31+) or human cardiac microvascular endothelial cells (HMVEC-C).

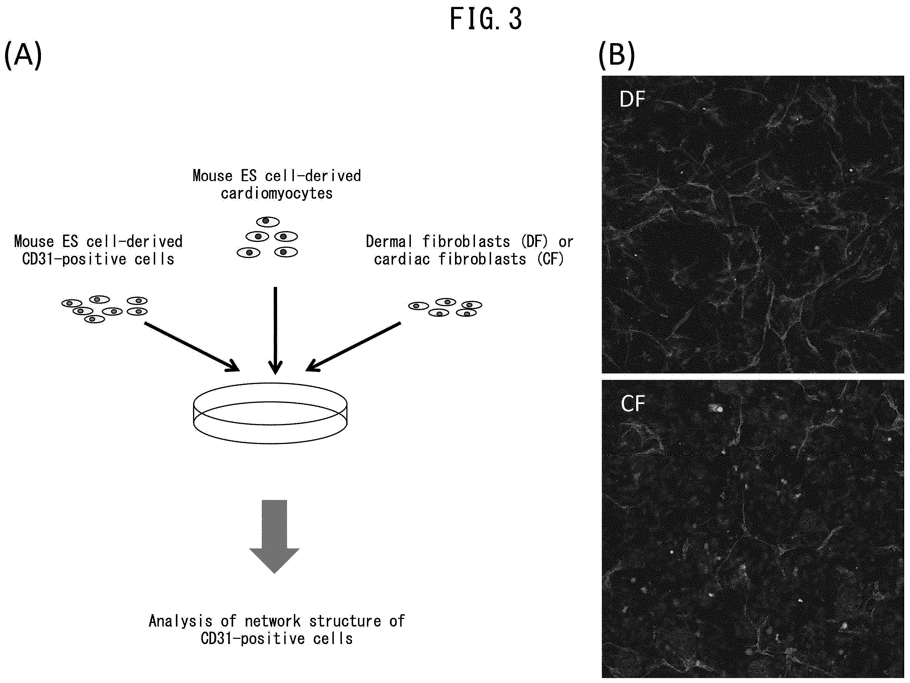

FIG. 3 depicts drawings indicating inhibition of vascular endothelial network formation by mouse cardiac fibroblasts. (A) is a drawing indicating the procedure of the present example. (B) indicates cardiomyocytes (green) and CD31-positive cells (red) following co-culturing of mouse dermal fibroblasts (DF) or mouse cardiac fibroblasts (CF) with mouse ES cell-derived cardiomyocytes or mouse ES cell-derived vascular endothelial cells.

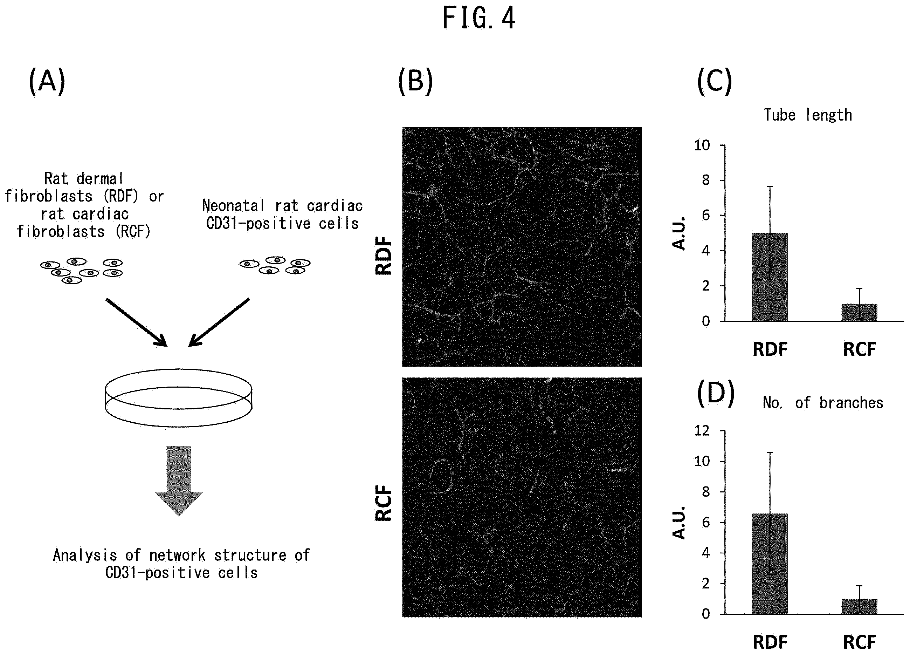

FIG. 4 depicts drawings indicating inhibition of vascular endothelial network formation by rat cardiac fibroblasts. (A) is a drawing indicating the procedure of the present example. (B) indicates vascular endothelial networks following co-culturing of neonatal rat dermal fibroblasts (RDF) or rat cardiac fibroblasts (RCF) with rat neonatal cardiac vascular endothelial cells. The green color represents CD31-positive cells and the blue color represents nuclei (Hoechst 33342). (C) depicts a graph indicating the total lengths of the vascular endothelial networks indicated in (B). (D) depicts a graph indicating the number of branches of the vascular endothelial networks indicated in (B).

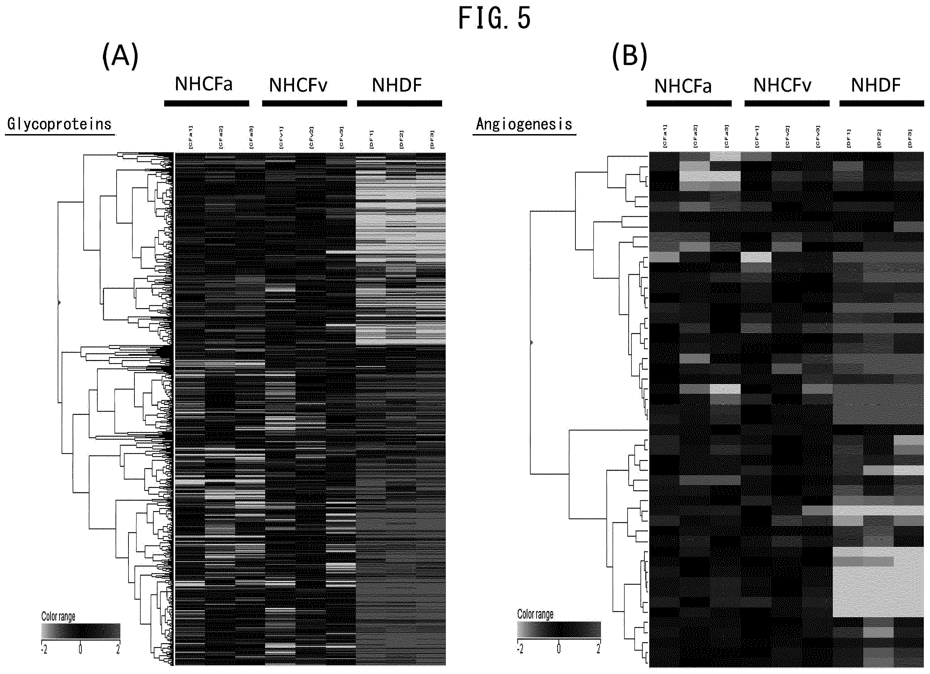

FIG. 5 depicts drawings comparing gene expression of dermal fibroblasts and cardiac fibroblasts. (A) indicates a heat map for glycoprotein-associated genes. (B) indicates a heat map for genes associated with angiogenesis.

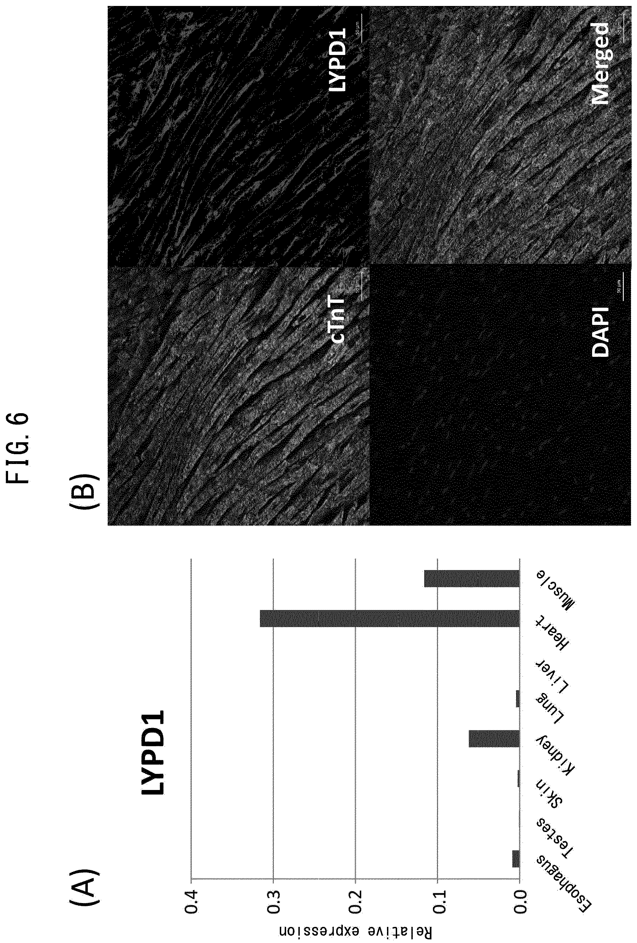

FIG. 6 depicts drawings indicating sites where LYPD1 is expressed. (A) is a graph obtained by evaluating the relative expression levels of LYPD1 in various rat organs as determined by qPCR. (B) indicates immunostaining images of rat cardiac tissue (cTnT: cardiac troponin T (green), LYPD1 (red), DAPI: nuclei (blue), Merged: merged).

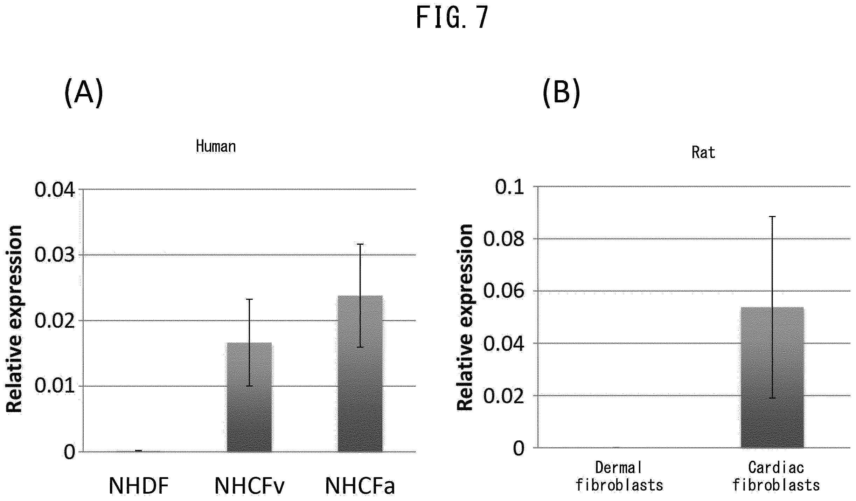

FIG. 7 depicts graphs comparing LYPD1 expression levels in human and rat primary cultured cells. (A) depicts a graph obtained by evaluating the relative expression levels of LYPD1 of primary normal human dermal fibroblasts (NHDF) and primary normal human cardiac fibroblasts (atrial fibroblasts represented by NHCF-a and ventricular fibroblasts represented by NHCF-v) by qPCR. (B) depicts a graph obtained by evaluating the relative expression levels of LYPD1 of primary rat dermal fibroblasts and primary rat cardiac fibroblasts by qPCR.

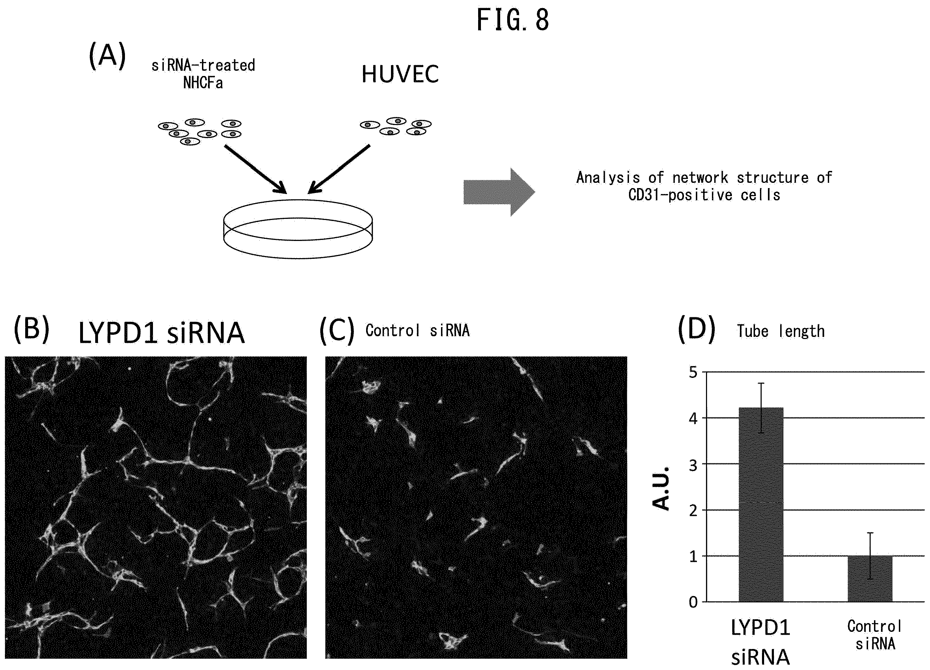

FIG. 8 depicts drawings indicating recovery of vascular network formation by inhibition of LYPD1 (siRNA). (A) is a drawing indicating the procedure of the present example. (B) indicates the results of immunostaining with anti-CD31 antibody after having introduced siRNA to LYPD1 into human cardiac fibroblasts followed by co-culturing with HUVEC. Green color represents CD31-positive cells. (C) indicates the results of immunostaining with anti-CD31 antibody after having introduced control siRNA into human cardiac fibroblasts followed by co-culturing with HUVEC. Green color represents CD31-positive cells. (D) is a graph indicating the total length of the vascular endothelial networks indicated in (B) and (C).

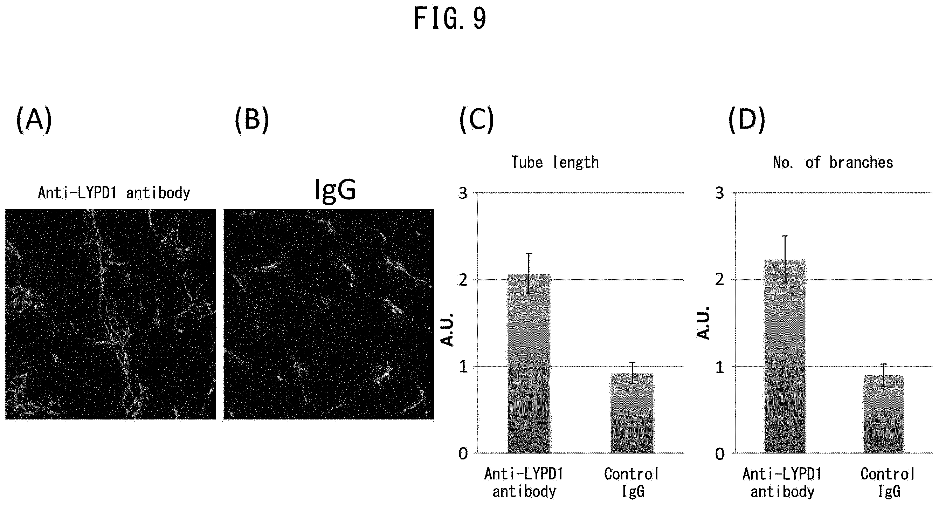

FIG. 9 depicts drawings indicating recovery of vascular network formation by inhibition of LYPD1 (with anti-LYPD1 antibody). (A) indicates the results of immunostaining with anti-CD31 antibody following co-culturing of human cardiac fibroblasts with HUVEC in the presence of anti-LYPD1 antibody. Green color represents CD31-positive cells. (B) indicates the results of immunostaining with anti-CD31 antibody following co-culturing of human cardiac fibroblasts with HUVEC in the presence of control IgG. Green color represents CD31-positive cells. (C) is a graph indicating the total length of the vascular endothelial networks indicated in (A) and (B). (D) is a graph indicating the number of branches of the vascular endothelial networks indicated in (A) and (B).

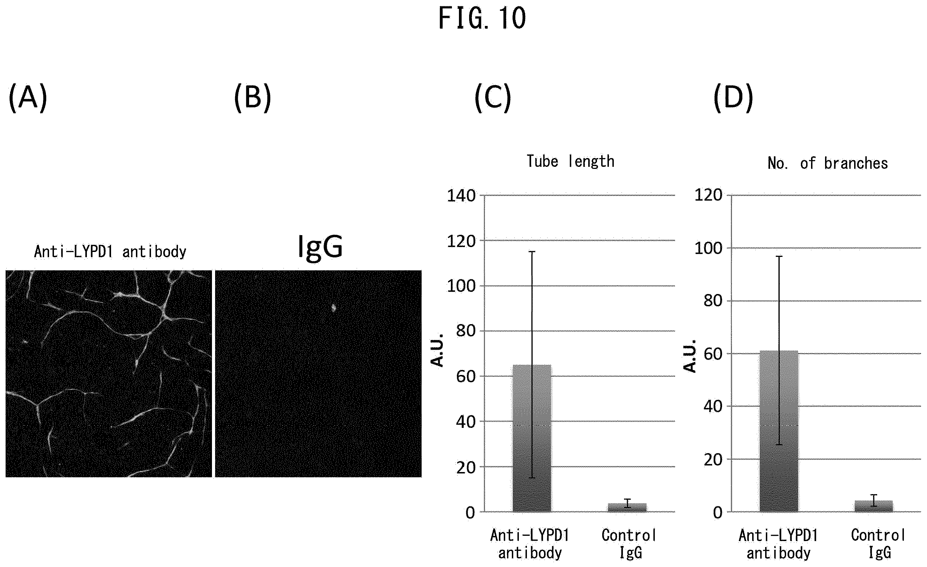

FIG. 10 depicts drawings indicating recovery of vascular network formation by inhibition of LYPD1 (with anti-LYPD1 antibody). (A) indicates the results of immunostaining with anti-CD31 antibody following co-culturing of rat neonatal cardiac fibroblasts with rat neonatal cardiac vascular endothelial cells in the presence of anti-LYPD1 antibody. Green color represents CD31-positive cells. (B) indicates the results of immunostaining with anti-CD31 antibody following co-culturing of rat neonatal cardiac fibroblasts with rat neonatal cardiac vascular endothelial cells in the presence of control IgG. Green color represents CD31-positive cells. (C) is a graph indicating the total length of the vascular endothelial networks indicated in (A) and (B). (D) is a graph indicating the number of branches of the vascular endothelial networks indicated in (A) and (B).

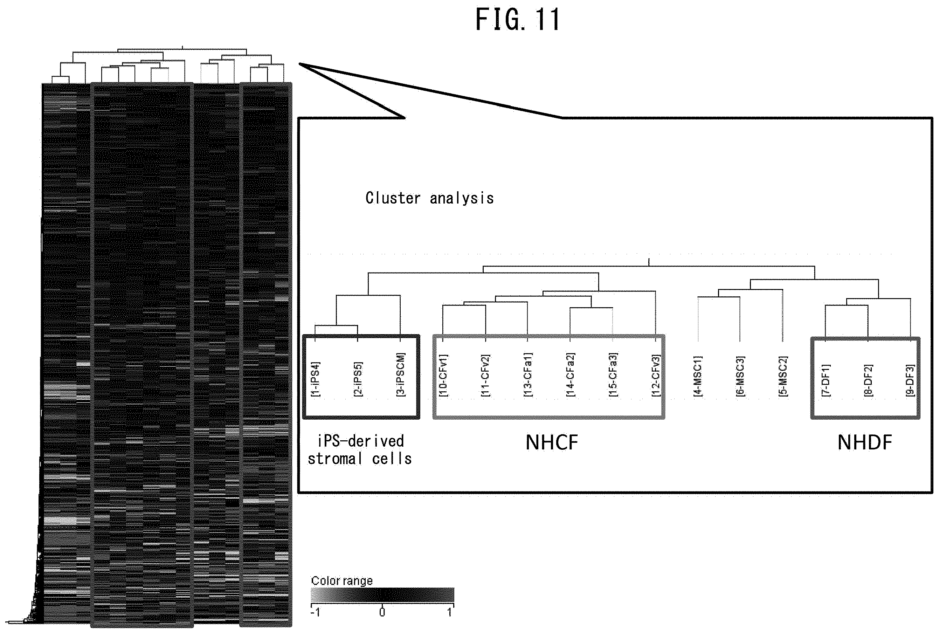

FIG. 11 is a drawing indicating the results of analyzing gene expression with a microarray in normal human dermal fibroblasts (NHDF) and normal human cardiac fibroblasts (NHCF), iPS-derived stromal cells and mesenchymal stem cells (MSC). A cluster analysis of the results is shown on the right.

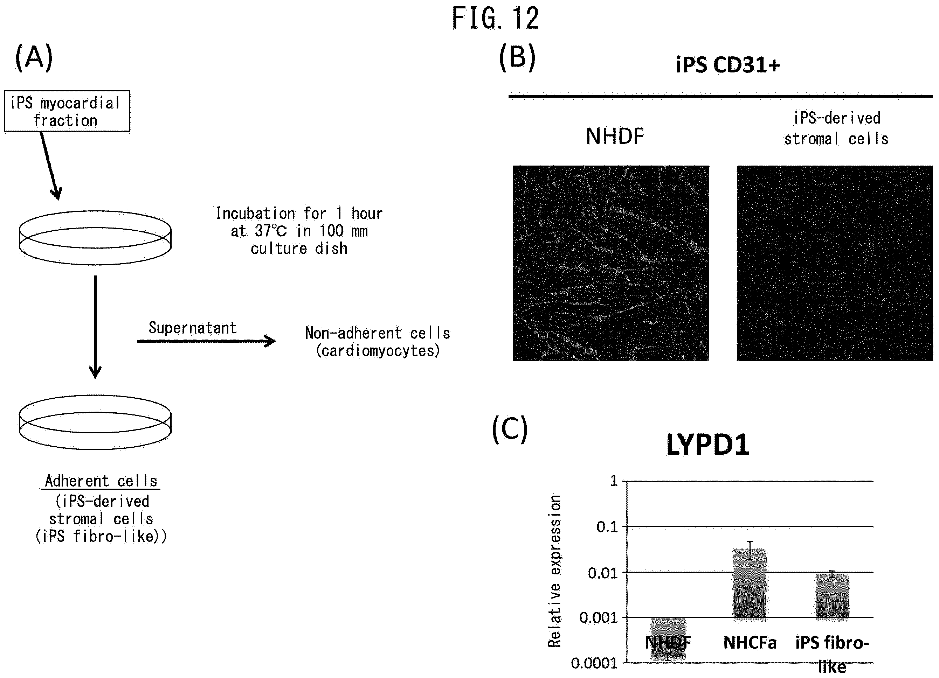

FIG. 12 depicts drawings indicating inhibition of vascular endothelial network formation derived from human iPS CD31-positive cells (iPS CD31+) by human IPS-derived stromal cells (iPS fibro-like). (A) is a drawing indicating the procedure of the present example. (B) depicts the results of immunostaining with anti-CD31 antibody following co-culturing of normal human dermal fibroblasts (NHDF) or human iPS-derived stromal cells with human iPS CD31-positive cells. Red color represents CD31-positive cells. (C) is a graph obtained by evaluating expression of LYPD1 in normal human dermal fibroblasts (NHDF), normal human cardiac fibroblasts (NHCFa) and human iPS-derived stromal cells (iPS fibro-like) by qPCR.

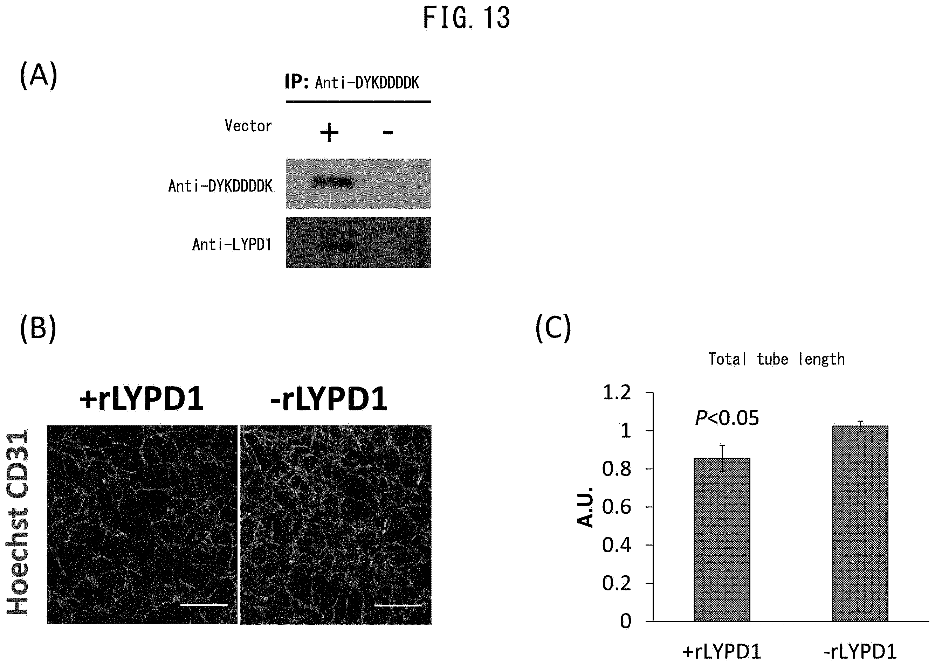

FIG. 13 depicts drawings indicating inhibition of vascular endothelial network formation by recombinant LYPD1. (A) indicates the results of applying FLAG-LYPD1 protein purified using anti-DYKDDDDK-tagged antibody magnetic beads to dodecyl sulfate-polyacrylamide gel electrophoresis and immunoblotting and detecting with peroxidase-bound anti-DYKDDDDK-tagged monoclonal antibody (top) and rabbit polyclonal antibody anti-LYPD1 antibody (bottom). (B) depicts the status of vascular endothelial network (tube) formation following treatment with recombinant LYPD1 protein. CD31 (green) and nuclei (Hoechst 33342 (blue)) were stained. The scale bars indicate 400 µm. (C) is a graph indicating the total length of the vascular endothelial networks (tubes) following treatment with recombinant LYPD1 protein. The total of the lengths of the tubes formed by CD31-positive cells were calculated. Values were calculated as the mean ± standard deviation from the results of three independent experiments. P < 0.05.

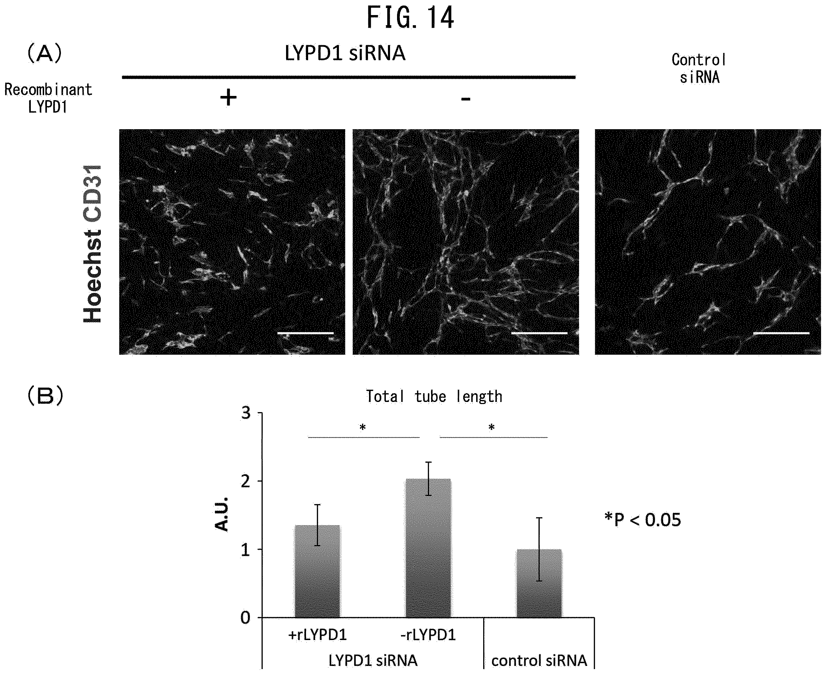

FIG. 14 depicts recovery of vascular endothelial network formation through suppression of LYPD1. (A) depicts the results of staining with CD31 antibody and Hoechst 33342 following co-culturing of control siRNA or normal human cardiac fibroblasts (2.4 × 10⁵ cells/cm²) introduced with LYPD1 siRNA with HUVEC (2 × 10⁴ cells/cm²) and fixing after culturing for 3 days. rLYPD1 was added at a concentration of 1.5 µg/mL. An equal amount of buffer (composition: 500 µg/mL DYKDDDDK peptide, 10 mM Tris-HCl (pH 7.4), 150 mM NaCl) was added in the case of -rLYPD1. The images were acquired using the ImageXpress Ultra Confocal High Content Screening System (Molecular Devices, LLC). Blue color represents nuclei (Hoechst 33342) and green color represents CD31. (B) indicates a graph of total tube length obtained by measuring the lengths of CD31-positive cells in the images acquired in (A) using MetaXpress software (Molecular Devices, LLC).

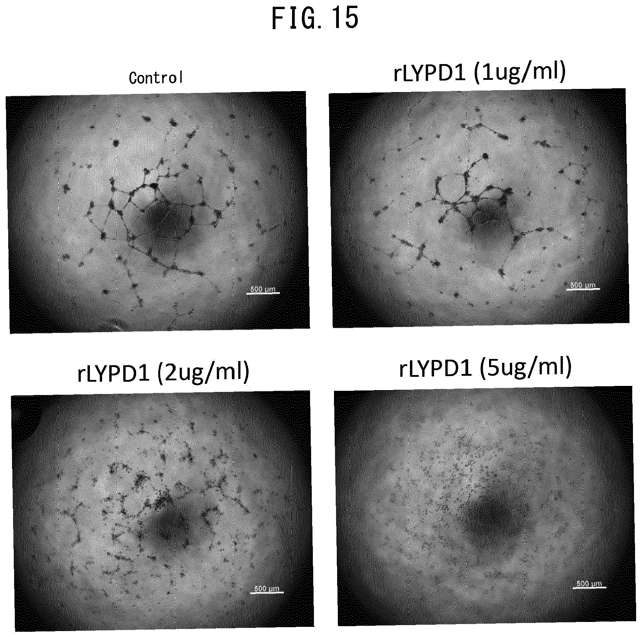

FIG. 15 depicts images indicating the effect of rLYPD1 on lumen formation of HUVEC on Matrigel ®.

### DESCRIPTION OF EMBODIMENTS

### 1-1. LYPD1 Protein

In the present specification, the term "LYPD1" is used with the same meaning as is generally used in the art and refers to protein also referred to as LY6/PLAUR domain-containing 1, PHTS or LYPDC1 (to be referred to as "LYPD1"). LYPD1 is a protein that is widely preserved in mammals and has been found in mammals such as humans, monkeys, dogs, cows, mice and rats. The mRNA and amino acid sequences of naturally-occurring human LYPD1 are provided in, for example, the GenBank database and GenPept database under accession numbers NM 001077427 (SEQ ID NO: 1), NP 001070895 (SEQ ID NO: 2), NM 144586 (SEQ ID NO: 3), NP 653187 (SEQ ID NO: 4), NM 001321234 (SEQ ID NO: 5), NP 001308163 (SEQ ID NO: 6), NM 001321235 (SEQ ID NO: 7) and NP 001308164 (SEQ ID NO: 8). In addition, the mRNA and amino acid sequences of naturally-occurring mouse LYPD1 are provided in, for example, the GenBank database and GenPept database under accession numbers NM 145100 (SEQ ID NO: 9), NP 659568 (SEQ ID NO: 10), NM 001311089 (SEQ ID NO: 11), NP 001298018 (SEQ ID NO: 12), NM 001311090 (SEQ ID NO: 13) and NP 001298019 (SEQ ID NO: 14).

In the present specification, the term "LYPD1" may include naturally-occurring LYPD1, mutants thereof and modified forms thereof. This term may also refer to a fusion protein obtained in which an LYPD1 domain retaining at least one type of LYPD1 activity is fused with another polypeptide, for example. Although the LYPD1 may be of any biological origin, it is preferably derived from a mammal (such as a human, primate other than a human, rodent (such as a mouse, rat, hamster or guinea pig), rabbit, dog, cow, horse, pig, cat, goat or sheep), more preferably derived from a human or primate other than a human, and is particularly preferably human LYPD1.

Although LYPD1 is known to be a protein that is highly expressed in the brain, very little is known about its function. LYPD1 is thought to be a glycosyl-phosphatidylinositol (GPI)-anchored protein based on the amino acid motif thereof.

During the course of conducting research on the construction of three-dimensional biological tissue using tissue engineering, the inventors of the present invention discovered a phenomenon by which vascular endothelial cell network formation is remarkably inhibited in the case of having co-cultured cardiac fibroblasts derived from mammals consisting of any of mice, rats and humans with vascular endothelial cells. As a result of a detailed investigation of the cause thereof, the inventors of the present invention found that vascular network hypoplasia is improved by inhibiting LYPD1. The present invention was completed on the basis of these findings.

### 1-2. Vascular Endothelial Network

In the present specification, the term "vascular endothelial network" refers to a capillary-like network constructed by vascular endothelial cells and/or vascular endothelial progenitor cells in biological tissue. CD31 protein is known to be a cell surface marker of vascular endothelial cells and/or vascular endothelial progenitor cells, and the presence of vascular endothelial cells and/or vascular endothelial progenitor cells in biological tissue can be detected by detecting CD31 protein using an arbitrary method. Vascular endothelial networks form a lumen structure and become vascular networks through which fluids, and particularly blood, pass. In order to exist in biological tissue, it is necessary for blood containing nutrients and oxygen to spread throughout the entire network, and in order to accomplish this, it is necessary to construct a highly dense vascular network. In biological tissue, the higher the density of the network structure of the vascular endothelial network, the greater the ability to transport blood and the like within the biological tissue, thus making high density preferable. Whether or not the LYPD1 inhibitor of the present invention promotes vascular endothelial network formation can be determined by evaluating the length and/or number of branches of a vascular endothelial network constructed in the manner described above. The length of a vascular endothelial network refers to the length obtained by combining vascular endothelial networks per unit area, while the number of branches of a vascular endothelial network refers to the total number of sites where vascular endothelial networks are connected that are present per unit area. Namely, LYPD1 inhibitors having a higher ability to promote vascular endothelial network formation the higher the length and/or number of branches of the vascular endothelial network in comparison with the case of not using the aforementioned LYPD1 inhibitor (or in the case of a compound serving as a negative control) when screening LYPD1 inhibitors are evaluated as LYPD1 inhibitors. The length and/or number of branches of a vascular endothelial network can be calculated using the CD31-positive regions of images acquired with a confocal fluorescence microscope and the like as vascular endothelial cells using, for example, MetaXpress software (Molecular Devices, LLC).

### 1-3. Biological Tissue

In the present specification, the term "biological tissue" refers to an arbitrary portion that composes a mammal, and typically refers to tissue composed by congregating two or more cells. In the present invention, "biological tissue" may be any portion of a subject, biological tissue sampled from a subject or biological tissue fabricated using tissue engineering techniques either outside the body (in vitro) or inside the body (in vivo). In the present specification, the term "subject" refers to a mammal such as a cow, horse, rodent (such as a rat or mouse), cat, dog or primate. A subject according to the present invention is preferably a human. In the present invention, biological tissue preferably contains vascular endothelial cells and/or vascular endothelial progenitor cells.

A known method can be used to fabricate biological tissue using tissue engineering techniques outside the body (in vitro) or inside the body (in vivo). For example, biological tissue obtained according to a method for constructing biological tissue by layering cell sheets on a vascular bed (see International Publication No. WO 2012/036224 and International Publication No. WO 2012/036225), a method for fabricating a three-dimensional structure using cells coated with an adhesive film (see Japanese Unexamined Patent Publication No. 2012-115254), or method for generating organs in the body (see Kobayashi, T. and Nakauchi, H.: "From cell therapy to organ regeneration therapy: generation of functional organs from pluripotent stem cells", Nihon Rinsho, 2011 Dec., 69(12), 2148-2155, International Publication No. WO 2010/021390, International Publication No. WO 2010/097459), as well as biological tissue obtained according to known production methods, can be applied to the present invention and are included within the scope of the present invention.

A "cell sheet" used when fabricating biological tissue using tissue engineering techniques outside the body (in vitro) refers to a cell population in the form of a single sheet or multilayered sheet obtained by culturing a cell population containing a plurality of arbitrary cells and detaching from the cell culture substrate. Examples of methods used to obtain cell sheets include a method in which cells are detached in the form of a sheet from a stimulus-responsive culture substrate while maintaining an adhered state among the cells by culturing the cells on a stimulus-responsive culture substrate coated with a polymer in which the molecular structure thereof changes in response to a stimulus such as temperature, pH or light and changing the surface of the stimulus-responsive culture substrate by changing the conditions of the stimulus such as temperature, pH or light, and a method for obtaining a cell sheet by culturing cells on an arbitrary culture substrate and physically detaching the cells with forceps and the like. A temperature-responsive culture substrate having the surface thereof coated with a polymer in which hydration force changes over a temperature range of 0°C to 80°C is known as a stimulus-responsive culture substrate for obtaining a cell sheet. Cells can be detached in the form of a sheet and subsequently recovered by culturing the cells on a temperature-responsive culture substrate over a temperature range for which hydration force of the polymer is weak followed by changing the temperature of the culture broth to a temperature at which the hydration force of the polymer becomes strong.

The temperature-responsive culture substrate used to obtain a cell sheet is preferably a substrate that causes the hydration force of the surface thereof to change over a temperature range that allows the cells to be cultured. That temperature range is typically the temperature at which the cells are cultured, and is preferably, for example, 33°C to 40°C. The temperature-responsive polymer coated onto the culture substrate used to obtain a cell sheet may be a homopolymer or copolymer. An example of such a polymer is the polymer described in Japanese Unexamined Patent Publication No. H2-211865.

The following provides an explanation using as an example the case of using poly(N-isopropylacrylamide) as a stimulus-responsive polymer, and particularly a temperature-responsive polymer. Poly(N-isopropylacrylamide) is known to be a polymer having a lower limit critical solution temperature at 31°C and undergoes dehydration in water at a temperature of 31°C or higher that causes the polymer chain to aggregate and form a suspension when in the free state. Conversely, the polymer chain is hydrated and dissolves in water at a temperature below 31°C. In the present invention, this polymer is immobilized by coated onto a Petri dish or other substrate surface. Thus, although the polymer on the surface of culture substrate is similarly dehydrated if the temperature is 31°C or higher, since the polymer chain is immobilized on the surface of the culture substrate, the surface of the culture substrate exhibits hydrophobicity. Conversely, although the polymer on the surface of the culture substrate is hydrated at a temperature below 31°C, since the polymer chain is coated on the surface of the culture substrate, the surface of the culture substrate exhibits hydrophilicity. Cells adhere to the surface of the culture substrate at this time resulting in a suitable surface that allows proliferation, while the hydrophilic surface prevents cells from being able to adhere thereto. Consequently, when the substrate is cooled to below 31°C, the cells detach from the substrate surface. If the cells are cultured to confluency on the whole culture surface, a cell sheet can be recovered by cooling the substrate to below 31°C. Although there are no limitations on the temperature-responsive culture substrate provided it has the same effect, UpCell® commercially available from CellSeed Inc. (Tokyo, Japan), for example, can be used.

The biological tissue used in the present invention may be a cell sheet obtained by layering multiple cell sheets (layered cell sheet). Examples of methods used to produce a layered cell sheet include a method comprising aspirating a cell sheet floating in a culture medium with a pipette and the like, releasing onto a different cell sheet in a culture dish and layering the cell sheets by liquid flow, and a method by which cell sheets are layered using a cell transfer tool. Biological tissue containing a layered cell sheet may also be obtained by other known methods.

### 2. LYPD1 Inhibitor

In the present specification, the term "LYPD1 inhibitor" is a term that is to be understood in the broad sense and refers to naturally-occurring or synthesized compounds or cells (such as cells provided in the form of a cell suspension or cell sheet) that exhibit a biological effect that directly and/or indirectly inhibits the activity of LYPD1 or significantly suppressed that activity. For example, LYPD1 inhibitors include selective LYPD1 inhibitors and LYPD1 expression inhibitors to be subsequently described. In particular, in the present invention, the LYPD1 inhibitor is a substance that promotes vascular endothelial network formation by acting directly and/or indirectly on LYPD1 expressed in biological tissue in which vascular endothelial network formation has been inhibited. In one embodiment thereof, the LYPD1 inhibitor of the present invention may be a pharmaceutically acceptable salt thereof. In the present specification, "pharmaceutically" or "pharmaceutically acceptable" refers to molecules and compositions that do not adverse side effects, allergic reactions or other harmful effects when properly administered to a mammal, and particularly a human. A pharmaceutically acceptable carrier or vehicle refers to a nontoxic, solid, semi-solid or liquid injection preparation, encapsulated substance or any type of formulation adjuvant. In addition, the LYPD1 inhibitor may contain cells that lowly express LYPD1 such as cells that express lower levels of LYPD1 than cardiac fibroblasts (examples of which include cells derived from the esophagus, testes, skin, kidney, lung, liver or muscle, preferably cells derived from the esophagus, testes, lung or liver, more preferably fibroblasts derived from the esophagus, testes, skin, lung or liver, and most preferably fibroblasts derived from the skin).

In the present specification, the term "selective LYPD1 inhibitor" refers to an inhibitor that selectively inhibits LYPD1 in comparison with an LYPD protein other than LYPD1 (such as LYPD2, LYPD3, LYPD4, LYPD5 or LYPD6). "Selective" refers to the Ki value of the inhibitor with respect to LYPD1 being 1/5, preferably 1/10, more preferably 1/25 and even more preferably 1/100 or less of the Ki value with respect to other proteins. The Ki value of an LYPD1 inhibitor can be measured using various methods known in the art. The selective LYPD1 inhibitor may be, for example, an organic small molecule, aptamer, antibody, antibody fragment or combination thereof.

### 2-1. Organic Small Molecule

In the present specification, the term "organic small molecule" refers to a molecule of a size of the same degree as that of organic molecules typically used in pharmaceuticals. The size of an organic small molecule used as an LYPD1 inhibitor able to be used in the present invention is preferably within the range of about 5000 Da or less, more preferably within the range of about 2000 Da or less and most preferably within the range of about 1000 Da or less. In the present invention, an organic small molecule used as an LYPD1 inhibitor refers to that which promotes vascular endothelial network formation in biological tissue by acting directly and/or indirectly on LYPD1, and can be selected using the screening method to be subsequently described.

### 2-2. Aptamer

In the present specification, the term "aptamer" refers to a synthetic DNA or RNA molecule or peptide molecule having the ability to specifically bind to a target substance, and can be chemically synthesized in vitro in a short amount of time. Aptamers used in the present invention are able to inhibit the activity of LYPD1 by binding to LYPD1. Aptamers used in the present invention can be obtained by repeatedly binding to a small molecule, protein, nucleic acid of various other types of molecular targets in vitro and selecting using the SELEX method (see Tuerk, C. and Gold, L.: Science, 1990, 249 (4968), 505-510; Ellington, A.D. and Szostak, J.W.: Nature, 1990, 346 (6287), 818-822; U.S. Patent No. 6,867,289, U.S. Patent No. 5,567,588, U.S. Patent No. 6,699,843). In the present invention, an aptamer used as an LYPD1 inhibitor may promote vascular endothelial network formation in biological tissue by acting directly and/or indirectly on LYPD1 or can be selected using the screening method to be subsequently described.

Nucleic acid aptamers able to be used in the present invention are preferably made to have a prolonged half-life by subjecting to molecular modification with a polyethylene glycol (PEG) chain and the like as necessary since they are rapidly degraded and removed by nucleases in the blood.

### 2-3. Antibody and Antibody Fragment

LYPD1 inhibitor able to be used in the present invention may be an antibody or antibody fragment capable of partially or completely inhibiting LYPD1 activity by binding to LYPD1. An antibody or antibody fragment to LYPD1 able to be used in the present invention may be any of human-derived antibody, mouse-derived antibody, rat-derived antibody, rabbit-derived antibody or goat-derived antibody provided it inhibits LYPD1 activity, or may further be any of polyclonal or monoclonal antibodies, complete or truncated antibodies (such as F(ab')2, Fab', Fab or Fv fragments), chimeric antibodies, humanized antibodies or fully human antibodies. In the present specification, an antibody fragment refers to an F(ab')2, Fab', Fab or scFv antibody fragment and can be obtained by treating with a protease enzyme or reducing depending on the case.

An antibody or antibody fragment able to be used in the present invention can be produced in accordance with known methods for producing antibodies or antiserum by using LYPD1 protein or a portion thereof as antigen. LYPD1 protein or a portion thereof can be prepared by known protein expression methods and purification methods. In addition, an antibody or antibody fragment able to be used in the present invention can be produced through use of the phage display method (see, for example, FEBS Letters, 1998, Vol. 441, p. 20-24). In this method, human-type antibody is expressed on the surface of a phage in the form of a coat protein that composes the phase by using a phage that incorporates a human antibody gene in cyclic single-stranded DNA.

In the present invention, the antibody or antibody fragment used as an LYPD1 inhibitor may be that which promotes vascular endothelial network formation in biological tissue by acting directly or indirectly on LYPD1, and can be selected using the screening method to be subsequently described.

### 3. LYPD1 Expression Inhibitor

The "expression inhibitor" used in the present invention refers to a naturally-occurring or synthesized compound that exhibits a biological effect that directly and/or indirectly inhibits or significantly suppresses gene expression. Thus, the "LYPD1 expression inhibitor" refers to a naturally-occurring or synthesized compound that exhibits a biological effect that directly and/or indirectly inhibits or significantly suppresses expression of a gene that encodes LYPD1 gene. In addition, the LYPD1 inhibitor of the present invention may be cells treated with a LYPD1 expression inhibitor in which LYPD1 expression has been inhibited.

Examples of LYPD1 expression inhibitors that can be applied include antisense RNA or DNA molecules, RNAi-inducing nucleic acids (such as small interfering RNA (siRNA) or small hairpin RNA (shRNA)), microRNA (miRNA), ribozymes, genome-editing nucleic acids and expression vectors thereof, and combinations thereof. In addition, an organic small molecule, aptamer, antibody, antibody fragment or combination thereof, which exhibits a biological effect that directly and/or indirectly inhibits or significantly suppresses expression of LYPD1 gene, can also be applied as an LYPD1 expression inhibitor. Moreover, the LYPD1 expression inhibitor may also be cells treated with the above-mentioned LYPD1 expression inhibitor.

### 3-1. Antisense RNA or DNA Molecule

The antisense RNA or DNA molecule used in the present invention refers to a molecule having a function that inhibits protein synthesis handled by sense RNA thereof by forming two strands consisting of RNA such as messenger RNA (mRNA) having a certain function (sense RNA) and antisense RNA having a complementary base sequence. In the present invention, an antisense oligonucleotide containing antisense RNA or DNA inhibits translation into protein by binding to the mRNA of LYPD1. As a result, the expression level of LYPD1 can be reduced and LYPD1 activity can be inhibited. Methods for synthesizing antisense RNA or DNA molecules are widely known in the art and can be used in the present invention.

### 3-2. RNAi-inducing Nucleic Acid

RNAi-inducing nucleic acid able to be used in the present invention refers to a polynucleotide capable of inducing RNA interference (RNAi) by being introduced into cells, and is normally RNA, DNA or a chimeric molecule of RNA and DNA containing 19 to 30 nucleotides, preferably 19 to 25 nucleotides and more preferably 19 to 23 nucleotides, and may be arbitrarily modified. RNAi may be formed to mRNA or may be RNA immediately after transcription prior to processing, or in other words, RNA containing an exon, intron, 3'-untranslated region and 5'-untranslated region. An RNAi method able to be used in the present invention may be made to induce RNAi by a technique such as (1) directly introducing short double-stranded RNA (siRNA) into cells, (2) incorporating small hairpin RNA (shRNA) into various expression vectors and introducing that vector into cells, or (3) inserting short double-stranded DNA corresponding to siRNA into a vector having two promoters arranged in opposing directions between the promoters to fabricate a vector that expresses siRNA and then introducing that vector into cells. The RNAi-inducing nucleic acid may contain an RNA fragment of LYPD1 or siRNA, shRNA or miRNA capable of inducing the function thereof, and these RNAi nucleic acids may be introduced directly using a liposome or may be introduced using an expression vector that induces these RNAi nucleic acids.

The RNAi-inducing nucleic acid to LYPD1 used in the present invention can be synthesized using commonly known chemical synthesis techniques based on the LYPD1 sequence targeted by the RNAi-inducing nucleic acid. For example, the RNAi-inducing nucleic acid can be synthesized chemically using an automated DNA (/RNA) synthesizer using DNA synthesis technology such as the solid phase phosphoramidite method, or can be synthesized by commissioning synthesis to commissioned synthesis company relating to siRNA (such as Life Technologies, Inc.). According to one embodiment of the present invention, the siRNA used in the present invention may be induced from a precursor thereof in the form of short-hairpin type double-stranded RNA (shRNA) through processing with intracellular RNase in the form of a dicer. In the present invention, the RNAi-inducing nucleic acid used is RNA derived from SEQ ID NO: 15 of the complementary sequence thereof (SEQ ID NO: 16) containing 19 to 30, preferably 19 to 25 and more preferably 19 to 23 contiguous nucleotides. This RNA can have one or a plurality of, such as two, additional sequences (such as tt, uu or tg) added to the 5'-end or 3'-end to prevent degradation within cells and enhance stability. The RNAi-inducing nucleic acid to LYPD1 used in the present invention is only required to exhibit a biological effect that inhibits or significantly suppresses expression of LYPD1, and can be synthesized with reference to the base sequence of LYPD1 by a person with ordinary skill in the art. For example, although LYPD1 containing the following sequences can be used as siRNA, this is not intended to be limiting, but rather sequences complementary to the following sequences may also be used.
5'-GGCUUUGCGCUGCAAAUCC-3' (SEQ ID NO: 15)
5'-GGAUUUGCAGCGCAAAGCC-3' (SEQ ID NO: 16)

### 3-3. MicroRNA (miRNA)

MicroRNA (miRNA) is a single-stranded RNA molecule 21 to 25 bases in length that is involved in regulation of expression following transcription of a gene in eukaryotes. miRNA typically suppresses protein production by recognizing the 3'-UTR of mRNA to suppress translation of target mRNA. Thus, miRNA capable of directly and/or indirectly reducing the expression level of LYPD1 is also included within the scope of the present invention.

### 3-4. Ribozyme

Ribozymes is the generic term of enzymatic RNA molecules capable of catalyzing specific cleavage of RNA. Although there are ribozymes having various activities, ribozymes that site-specifically cleave RNA have come to be able to be designed through research focusing on ribozymes functioning as enzymes that cleave RNA in particular. Although there are group I intron type ribozymes and ribozymes of a size of 400 nucleotides or more in the manner of M1 RNA contained in RNase P, there are also ribozymes having an active domain of about 40 nucleotides referred to as hammerhead or hairpin ribozymes (see, for example, Koizumi, M. and Ohtsuka, E.: Protein and Nucleic Acid Enzymes, 1990, 35, 2191).

For example, although the self-cleaving domain of hammerhead ribozymes cleaves the 3'-side of C15 of the sequence G13U14C15, the formation of a base pair between U14 and A9 is considered to be important for the activity thereof, and has been indicated as being also able to cleave A15 or U15 instead of C15 (see, for example, Koizumi, M., et al.: FEBS Lett., 1988, 228, 228). If a ribozyme is designed that is complementary to an RNA sequence in which the substrate binding site is close to the target site, a ribozyme that cleaves RNA using a restrictase can be obtained that recognizes the sequence UC, UU or UA in the target RNA, and can be produced by a person with ordinary skill in the art with reference to the following references: Koizumi, M., et al.: FEBS Lett., 1988, 239, 285; Koizumi, M. and Ohtsuka, E.: Protein and Nucleic Acid Enzymes, 1990, 35, 2191; Koizumi, M., et al.: Nucl. Acids Res., 1989, 17, 7059).

In addition, a hairpin ribozyme can also be used in the present invention. This type of ribozyme is found in, for example, the minus strand of satellite RNA of tobacco ringspot virus (Buzayan, J.M.: Nature, 1986, 323, 349). Target-specific RNA-cleaving ribozymes have also been indicated to be able to be fabricated from hairpin ribozymes (see, for example, Kikuchi, Y. and Sasaki, N.: Nucl. Acids Res., 1991, 19, 6751; Kikuchi, Y.: Chemistry and Biology, 1992, 30, 112). Expression of LYPD1 gene can be inhibited by specifically cleaving the transcription product of a gene encoding LYPID1 using a ribozyme. Thus, ribozymes targeted at LYPD1 are also included within the scope of the present invention.

### 3-5. Genome-editing Nucleic Acid

A genome-editing nucleic acid, which exhibits a biological effect of directly and/or indirectly inhibiting or significantly suppressing expression of LYPD1 gene can be used as an LYPD1 expression inhibitor in one embodiment of the present invention. In the present specification, a genome-editing nucleic acid refers to a nucleic acid used to edit a desired gene in a system that uses nuclease used in gene targeting. Nucleases used in gene targeting include known nucleases as well as novel nucleases to be used for gene targeting in the future. Examples of known nucleases include CRISPR/Cas9 (Ran, F.A., et al.: Cell, 2013, 154, 1380-1389), TALEN (Mahfouz, M., et al.: PNAS, 2011, 108, 2623-2628), and ZEN (Urnov, F., et al.: Nature, 2005, 435, 646-651).

The following provides an explanation of the CRISPR/Cas9 system using CRISPR/Cas9 able to be used in one embodiment of the present invention.

The CRISPER/Cas9 system allows double strand cleavage to be introduced into an arbitrary site of DNA. At least three elements consisting of protospacer adjacent motif (PAM sequence), a guide RNA (gRNA) and a Cas protein (Cas, Cas9) are required to use the CRISPR/Cas9 system.

The gRNA is designed so as to form a sequence complementary to a target site adjacent to the PAM sequence (5'-NGG) followed by introduction thereof into desired cells along with the Cas protein. The introduced gRNA and Cas protein form a complex. The gRNA sequence-specifically binds to the genome and the Cas protein cleaves the two strands of the target genomic DNA using the nuclease activity thereof.

Subsequently, homology directed repair (HDR) or non-homologous end joining (NHEJ) occur in the cells that have been subjected to double-strand cleavage by nuclease. In the case a suitable DNA fragment (such as a template for HDR repair) is present within the cells, homologous recombination occurs and modification such as deletion, insertion or destruction can be carried out in an arbitrary genome. In the case a template for HDR repair is not present, there are cases in which deletion or addition of multiple bases may occur during the course of NHEJ. As a result, a frame shift occurs in the region encoding protein and the protein reading frame collapses or a premature stop codon is introduced, and as a result thereof, a desired protein can be knocked out.

In one embodiment of the present invention, the genome-editing nucleic acid may be gRNA targeting LYPD1 gene or a vector expressing that gRNA. In another embodiment, the genome-editing nucleic acid may further contain a nucleic acid expressing a nuclease used in gene targeting. The gRNA and nuclease used in gene targeting (preferably Cas protein) may be encoded in the same vector or vectors may be used in which they are encoded separately. In another embodiment, the genome-editing nucleic acid may further contain a template nucleic acid for HDR repair. The genome-editing nucleic acid may be a plasmid vector or viral vector. A widely known method can be used for the method used to introduce into arbitrary cells according to the genome-editing nucleic acid and there are no particular limitations thereon.

### 3-6. Organic Small Molecules, Aptamers, Antibodies and Antibody Fragments as LYPD1 Expression Inhibitors

In one embodiment of the present invention, the LYPD1 expression inhibitor is provided in the form of an organic small molecule, aptamer, antibody, antibody fragment or combination thereof that exhibits a biological effect that directly and/or indirectly inhibits or significantly suppresses expression of LYPD1 gene. Examples of such substances that can be used include NF-κB inhibitors. NF-κB inhibitors in the form of parthenolide derivatives, and particularly dimethylaminoparthenolide (DMAPT), are known to suppress expression of LYPD1 (Burnett, R.M., et al.: Oncotarget, 6, 12682-12696 (2015)). Namely, in one embodiment, the LYPD1 inhibitor of the present invention may be one of the following parthenolide derivatives, although not limited thereto: 11βH, 13-dimethylamino parthenolide (DMAPT); 11βH, 13-diethylamino parthenolide; 11βH, 13-(tert-butylamino)parthenolide; 11βH, 13-(pyrrolidin-1-yl)parthenolide; 11βH, 13-(piperidin-1-yl)parthenolide; 11βH, 13-(morpholin-1-yl)parthenolide; 11βH, 13-(4-methylpiperidin-1-yl)parthenolide;11βH, 13-(4-methylpiperazin-1-yl)parthenolide; 11βH, 13-(homopiperidin-1-yl)parthenolide; 11βH, 13-(heptamethyleneimin-1-yl)parthenolide; 11βH,13-(azetidin-1-yl)parthenolide; 11βH, 13-diallylamino parthenolide and pharmaceutically acceptable salts thereof. These parthenolide derivatives able to able to be used in one embodiment of the present invention can be obtained by referring to International Publication No. WO 2005/007103 and are included within the scope of the present invention.

### 3-7. Expression Vector of LYPD1 Expression Inhibitor

In one embodiment of the present invention, the LYPD1 expression inhibitor used as an LYPD1 inhibitor may be provided as an expression vector in which the previously described antisense RNA or DNA molecule, RNAi-inducing nucleic acid, microRNA (miRNA), ribozyme or genome-editing nucleic acid is encoded in an arbitrary vector. In the present invention, there are no particular limitations on the vector used to express the LYPD1 expression inhibitor and a known vector can be suitably selected. Examples thereof include a plasmid vector, a cosmid vector, a fosmid vector, a viral vector and an artificial chromosome vector. Introduction using a known gene engineering technology can be used for the method used to introduce the LYPID1 expression inhibitor into the vector and there are no particular limitations thereon.

### 3-8. Cells Treated with LYPD1 Expression Inhibitor

In one embodiment of the present invention, the LYPD1 inhibitor may also constitute cells treated with an expression vector in which the previously described antisense RNA or DNA, RNAi-inducing nucleic acid, microRNA (miRNA), ribozyme or genome-editing nucleic acid are encoded in an arbitrary vector. In addition, in one embodiment of the present invention, the LYPD1 inhibitor may also be cells in which have been introduced an expression vector of an LYPID1 expression inhibitor as a result of having treated the cells with an expression vector of the LYPD1 expression inhibitor. The method used to introduce an expression vector of the LYPID1 expression inhibitor into cells may be in accordance with a known method and there are no particular limitations thereon. In addition, there are also no particular limitations on the method used to select cells introduced with the expression vector that temporarily or continuously express the LYPD1 expression inhibitor, and for example, a drug (such as neomycin or hygromycin) corresponding to a drug resistance gene encoded by an expression vector is selected for such use.

In addition, in one embodiment of the present invention, the LYPD1 inhibitor may be cells treated with the previously described organic small molecule, aptamer, antibody or antibody fragment.

### 4. Pharmaceutical Composition

The present invention may also be a pharmaceutical composition for treating and/or preventing angiogenic disorders, comprising the LYPD1 inhibitor as an active ingredient thereof.

The pharmaceutical composition comprising as active ingredient the LYPD1 inhibitor or LYPD1 expression inhibitor used in the present invention promotes vascular endothelial network formation in biological tissue expressing LYPD1 such as biological tissue of the brain, heart, kidney or muscle that highly expresses LYPD1. As a result, the pharmaceutical composition comprising the LYPD1 inhibitor or LYPD1 expression inhibitor as an active ingredient thereof is able to treat and/or prevent angiogenic disorders by promoting vascular endothelial network formation. Examples of angiogenic disorders able to be treated and/or prevented by the pharmaceutical composition comprising as an active ingredient thereof the LYPD1 inhibitor or LYPD1 expression inhibitor of the present invention include cerebrovascular disease, cerebral infarction, transient ischemic attack, moyamoya disease, angina, (peripheral) arterial occlusion, arteriosclerosis, Buerger's disease, myocardial infarction, ischemia, cardiomyopathy, congestive heart failure, coronary artery disease, hereditary hemorrhagic telangiectasia, ischemic heart disease, vascular intimal thickening, vascular occlusion, atherosclerotic peripheral vascular disease, portal hypertension, rheumatic heart disease, hypertension, thromboembolism, atherosclerosis, post-angioplasty restenosis, pulmonary arterial hypertension, vein graft disease, hypertensive heart disease, valvular heart disease, Kawasaki disease, dilated cardiomyopathy, hypertrophic cardiomyopathy, sarcoidosis, systemic scleroderma, aortitis syndrome, asymptomatic myocardial ischemia, internal carotid artery stenosis, vertebral artery stenosis, hemodialysis cardiomyopathy, diabetic cardiomyopathy, pulmonary arterial pulmonary hypertension, ischemic cardiomyopathy, post-coronary artery bypass surgery, post-percutaneous transluminal coronary angioplasty, acute myocardial infarction, subacute myocardial infarction, old myocardial infarction, exertional angina, unstable angina, acute coronary syndrome, coronary vasospastic angina, aortic valve stenosis, aortic valve insufficiency, mitral valve insufficiency and mitral valve stenosis.

In one embodiment, the pharmaceutical composition of the present invention may further comprise an angiogenesis induction factor. Examples of angiogenesis induction factors include vascular endothelial growth factor (VEGF), hepatocyte growth factor (HGF), fibroblast growth factor (FGF), epidermal growth factor (EGF), platelet-derived growth factor (PDGF), insulin-like growth factor (IGF), angiopoietin, transforming growth factor-β (TGF-β), placental growth factor (PIGF), matrix metalloproteinase (MMP) and family proteins thereof. One of these angiogenesis induction factors may be selected from among the above-mentioned factors or two or more be used in combination.

The pharmaceutical composition comprising as active ingredient thereof the LYPD1 inhibitor or LYPD1 expression inhibitor of the present invention is able to treat and/or prevent angiogenic disorders by applying to a subject requiring such.

A selective LYPD1 inhibitor can be administered in the form of a pharmaceutical composition as is defined below.

The LYPD1 inhibitor is preferably administered to a subject in a therapeutically effective amount. A "therapeutically effective amount" refers to an amount of the LYPD1 inhibitor that is required and sufficient for demonstrating the desired effect of treating and/or preventing angiogenic disorders.

The daily amount used of the LYPD1 inhibitor included in the present invention is determined within a range at the medical discretion of a physician. The therapeutically effective amount changes according to the disorder targeted for treatment and/or prevention and the severity of that disorder, activity of the compound used, composition used, patient age and body weight, patient health status, gender and diet, administration period, administration route and excretion rate of compound used, treatment period, concomitantly used drugs and other factors widely known in the field of health care. For example, initiating administration of LYPD1 inhibitor in an amount lower than the amount required for realizing a desired therapeutic effect and then gradually increasing the amount until the desired effect is realized is within the scope of that which can be realized by a person with ordinary skill in the art. The dose of the LYPD1 inhibitor can be altered over a broad range of 0.01 mg to 1000 mg per day in an adult. The pharmaceutical composition comprising the LYPD1 inhibitor as an active ingredient thereof preferably contains 0.01 mg, 0.05 mg, 0.1 mg, 0.5 mg, 1.0 mg, 2.5 mg, 5.0 mg, 10.0 mg, 15.0 mg, 25.0 mg, 50.0 mg, 100 mg, 250 mg or 500 mg of the active ingredient in order to administer according to the symptoms of the patient being treated. The pharmaceutical composition normally contains about 0.01 mg to about 500 mg of active ingredient and preferably contains about 1 mg to about 100 mg of active ingredient. The effective amount of drug is supplied at a dose of 0.0002 mg/kg of body weight to about 20 mg/kg of body weight, and particularly about 0.001 mg/kg of body weight to 7 mg/kg of body weight, per day.

### 5. Method for Producing Biological Tissue in which Vascular Endothelial Network Formation is Promoted

Vascular endothelial network formation is promoted in biological tissue by applying the LYPD1 inhibitor of the present invention. As a result, a functional vascular network is constructed in the biological tissue and a three-dimensional biological tissue having a certain degree of thickness can be obtained.

In one embodiment, the present invention provides a method for producing biological tissue in which vascular endothelial network formation has been promoted. This method comprises the following steps:
(a1) a step for providing a cell population containing first cells expressing LYPD1 and vascular endothelial cells and/or vascular endothelial progenitor cells,
(a2) a step for treating the cell population obtained in step (a1) with an LYPD1 inhibitor, and
(a3) a step for culturing the cell population obtained in step (a2); or
(b1) a step for treating a cell population containing first cells expressing LYPD1 with an LYPD1 inhibitor,
(b2) a step for contacting vascular endothelial cells and/or vascular endothelial progenitor cells with the cell population obtained in step (b1), and
(b3) a step for culturing the cell population obtained in step (b2).

The first cells expressing LYPD1 refer to cells having activity that inhibits vascular endothelial network formation by expressing LYPD1, and for example, are cells derived from biological tissue expressing LYPD1, preferably cells present in biological tissue of the brain, heart, kidney or muscle highly expressing LYPD1, and particularly preferably stromal cells or fibroblasts present in biological tissue of the brain, heart, kidney or muscle. The first cells expressing LYPD1 may also be cells induced from pluripotent stem cells. In the present invention, pluripotent stem cells refer to cells having self-replicability and pluripotency and are provided with the ability to form all types of cells that compose the body (pluripotency). Self-replicability refers to the ability to create two undifferentiated cells the same as itself from a single cell. The pluripotent stem cells used in the present invention include, for example, embryonic stem cells (ES cells), embryonic carcinoma cells (EC cells), trophoblast stem cells (TS cells), epiblast stem cells (EpiS cells), multipotent germline stem cells (mGS cells), and induced pluripotent stem cells (iPS cells). A method for inducting differentiation of these pluripotent stem cells can be carried out in accordance with, for example, the method of Matsuura et al. (Matsuura, K., et al.: Creation of human cardiac cell sheets using pluripotent stem cells, Biochem. Biophys. Res. Commun., 2012 Aug. 24, 425(2), 321-327).

The vascular endothelial cells and/or vascular endothelial progenitor cells able to be used in the present invention can be used provided they are cells that form blood vessels, and for example, human umbilical vein endothelial cells (HUVEC), human cardiac microvascular endothelial cells (HMVEC-C), pluripotent stem cell-derived vascular endothelial cells or vascular endothelial cells and/or vascular endothelial progenitor cells of mammals other than humans can be used. In the case the subject to which the cells are to be applied is a human, the cells are preferably human vascular endothelial cells and/or vascular endothelial progenitor cells.

In one embodiment, the "cell population containing first cells expressing LYPD1 and vascular endothelial cells and/or vascular endothelial progenitor cells" of the step (a1) refer to:
i) a cell population containing at least the first cells and at least vascular endothelial cells and/or vascular endothelial progenitor cells,
ii) biological tissue derived from a subject containing a cell population at least the first cells and at least vascular endothelial cells and/or vascular endothelial progenitor cells, or
iii) biological tissue fabricated using tissue engineering techniques containing a cell population containing at least the first cells and at least vascular endothelial cells and/or vascular endothelial progenitor cells.

Biological tissue in which vascular endothelial network formation has been promoted can be produced by treating the above-mentioned cell population obtained in step (a1) with LYPD1 inhibitor (step (a2)) and culturing for several days (such as for 1 day, 2 days, 3 days, 4 days or 5 days or more) (step (a3)). Namely, in the present embodiment, the method consists of treating with the LYPD1 inhibitor in a state in which the first cells expressing LYPD1 are present together with vascular endothelial cells and/or vascular endothelial progenitor cells. The culture period in step (a3) is suitably altered according to the number of cells, cell density, type of cells and the like.

In one embodiment, the method for producing biological tissue in which vascular endothelial network formation has been promoted is a method by which a cell population containing first cells expressing LYPD1 is treated with LYPD1 inhibitor (step (b1)) prior to co-culturing with vascular endothelial cells and/or vascular endothelial progenitor cells. Biological tissue in which vascular endothelial network formation has been promoted can be produced by contacting vascular endothelial cells and/or vascular endothelial progenitor cells with the cell population obtained in step (b1) (step (b2)) and culturing for several days (such as 1 day, 2 days, 3 day, 4 days or 5 days or more (step (b3)). The culture period in step (a3) is suitably altered according to the number of cells, cell density, type of cells and the like.

In the present invention, "treating with an LYPD1 inhibitor" refers to inhibiting the activity of LYPD1 by allowing the above-mentioned LYPD1 inhibitor to act on LYPD1 or LYPD1 gene (such as mRNA) expressed in the first cells according to a known method. Examples of methods that can be applied include a method consisting of culturing in medium containing the LYPD1 inhibitor, a method consisting of exposing to antisense RNA or DNA molecule, RNAi-inducing gene, miRNA, ribozyme, or expression vector or viral vector containing the same (such as a retroviral vector, adeno-associated viral vector, adenoviral vector or lentiviral vector), and a method consisting of introducing the LYPD1 inhibitor (such as an antisense RNA or DNA molecule, RNAi-inducing nucleic acid, miRNA, ribozyme or expression vector thereof) using the calcium phosphate method, electroporation, microinjection or lipofection. The optimum method is selected corresponding to the type and properties of the LYPD1 inhibitor.

In one embodiment, "treating with an LYPD1 inhibitor" may be a method by which second cells, in which the expression level of LYPD1 is lower than the expression level of LYPD1 of the first cells or is not expressed at all, are mixed or contact with the first cells and cultured. For example, a method may be employed by which the first cells and the second cells may be mixed and then cultured, or a method may be employed in which a cell population containing the first cells and a cell population containing the second cells are respectively formed into sheet-like cells (cell sheets) after which these cell sheets are contacted by layering. The ratio of the first cells to the second cells may be, for example, 199:1, 99:1, 95:5, 90:10, 80:20, 70:30, 60:40, 50:50, 40:60, 30:70, 20:80, 10:90, 5:95, 1:99 or 1:199, and there are no particular limitations thereon. The ratio is suitably altered corresponding to, for example, the type and LYPD1 expression level of the cells used.

Moreover, in one embodiment, the method of the present invention may be a method consisting of perfusion culturing in medium containing LYPD1 inhibitor. As a result, LYPD1 inhibitor is supplied continuously and the formation of a vascular endothelial network is promoted.

In one embodiment, the second cells are cells derived from, for example, skin, esophagus, lung and/or liver. These cells have a lower LYPD1 expression level in comparison with cells derived from heart, muscle, kidney or brain. The second cells are preferably skin-derived fibroblasts.

In one embodiment, the LYPD1 expression level of the second cells, having a LYPD1 expression level that is lower than the LYPD1 expression level of the first cells or do not express LYPD1 at all, is 1/2 or less, preferably 1/5 or less, more preferably 1/10 or less and even more preferably 1/50 or less than that of the first cells. In the present invention, LYPD1 expression level can be evaluating using a known technique such as quantitative PCR (qPCR), western blotting, flow cytometry (FACS), ELISA or an immunohistochemical method.

### 6. Use of LYPD1 Inhibitor to Produce Pharmaceutical Composition

In one embodiment, the LYPD1 inhibitor of the present invention can be used to produce a pharmaceutical composition for treating and/or preventing angiogenic disorders.

### 7. Method for Screening LYPD1 Inhibitors

The LYPD1 inhibitor of the present invention can be further used to identify LYPD1 inhibitors from among candidate substances by applying a well-known screening method. An example of such a method is described below.

According to the screening method, an LYPD1 inhibitor can be selected by evaluating binding of a candidate compound to LYPD1, a cell or cell membrane having LYPD1, or a fusion protein thereof, based on a label directly or indirectly bound to the candidate compound. Alternatively, according to the screening method, an LYPD1 inhibitor can be selected by measuring, qualitatively detecting or quantitatively detecting competitive binding to LYPD1 for a competing substance labeled with a candidate compound (such as an inhibitor or substrate).

For example, vector/host cells can be used in which an expression vector inserted with LYPD1 cDNA has been introduced into the host cells. For example, a baculovirus/Sf9 insect cell system, retrovirus/mammalian cell system or expression vector/mammalian cell system can be used. Examples of cells that can be used include, but are not limited to, HeLa, HepB3, LLC-PK1, MDCKII, CHO and HEK293 cells. In addition, in the LYPD1 inhibitor screening method of the present invention, cells derived from tissue that highly expresses LYPD1, such as cells derived from the brain, heart, muscle or kidney, and particularly cardiac fibroblasts, can also be used.

The LYPD1 inhibitor used in the present invention can be selected by pre-incubating cells or cells highly expressing LYPD1 obtained as previously described (at, for example, 2.4 × 10⁵ cells/cm²), vascular endothelial cells and/or vascular endothelial progenitor cells that construct vascular network (at, for example, 2.0 × 10⁴ cells/cm²), and a candidate substance, seeding into a culture dish and culturing for several days at 37°C and 5% CO₂, observing the vascular endothelial network formed by the vascular endothelial cells and/or vascular endothelial progenitor cells with a microscope (and preferably a fluorescence microscope), and evaluating the length and number of branches of the vascular endothelial network. The candidate substance may be mixed with the cells or cells highly expressing LYPD1 obtained as previously described and vascular endothelial cells and/or vascular endothelial progenitor cells constructing a vascular network followed by adding to a preliminarily seeded cell population and culturing.

The vascular endothelial network formed by the vascular endothelial cells and/or vascular endothelial progenitor cells may be evaluated by detecting using fluorescently labeled anti-CD31 antibody or vascular endothelial cell-specific antibody. In addition, the vascular endothelial network may also be evaluated by detecting fluorescence using vascular endothelial cells and/or vascular endothelial progenitor cells expressing a fluorescent protein such as GFP.

In one embodiment, the method for screening LYPD1 inhibitors may include, for example, the following steps:
(i-1) a step for providing a cell population containing first cells expressing LYPD1 and vascular endothelial cells and/or vascular endothelial progenitor cells,
(i-2) A step for treating the cell population obtained in step (i-1) with a candidate substance,
(i-3) a step for culturing the cell population obtained in step (i-2), and
(i-4) a step for evaluating formation of a vascular endothelial network in the cell population obtained in step (i-3); or,
(ii-1) a step for treating a cell population containing first cells expressing LYPD1 with a candidate substance,
(ii-2) a step for contacting vascular endothelial cells and/or vascular endothelial progenitor cells with the cell population obtained in step (ii-1),
(ii-3) a step for culturing the cell population obtained in step (ii-2), and
(ii-4) a step for evaluating formation of a vascular endothelial network in the cell population obtained in step (ii-3).

Cells highly expressing LYPD1 derived from the heart, muscle, kidney or brain, for example, may be used for the first cells able to be used in the present embodiment. In addition, cells introduced with a vector expressing LYPD1 may also be used.

In addition, in one embodiment, the method for screening LYPD1 inhibitors may consist of carrying out a step for treating, for example, cells comparatively highly expressing LYPD1 derived from the heart, muscle, kidney and/or brain with a candidate substance and selecting a candidate substance that lowers expression of LYPD1, or may be combined with the previously described method. Expression of LYPD1 can be detected using a known method, and can be detected using a known technique such as quantitative PCR (qPCR), western blotting, flow cytometry (FACS), ELISA or an immunohistochemical method.

### EXAMPLES

Although the following provides a more detailed explanation of the present invention based on examples thereof, these examples do not limit the present invention in any way.

### <Cells Used and Preparation Method>

The cells used in the following examples were as indicated below.
* Normal human dermal fibroblasts (purchased from Lonza, NHDF-Ad normal human dermal fibroblasts (CC-2511)
* Normal human cardiac fibroblasts (purchased from Lonza, NHCF-a (normal human cardiac fibroblasts-atrial (CC-2903)), NHCF-v (normal human cardiac fibroblasts-ventricular (CC-2904))
* Human umbilical vein endothelial cells (HUVEC) (purchased from Lonza, Cat. No. C2517A)
* Human cardiac microvascular endothelial cells (HMVEC-c) (purchased from Lonza, Cat. No. CC-7030)
* Human induced pluripotent stem cells: Fibroblasts are obtained by isolating a cell population exhibiting higher adhesion to the culture dish than cardiomyocytes from cell populations obtained when inducing differentiation of cardiomyocytes from human iPS cells. The cell population was designated as human iPS-derived stromal cells (see FIG. 12(A)). Differentiation to cardiomyocytes from human iPS cells was carried out according to the method described in Matsuura, K., et al.: Creation of human cardiac cell sheets using pluripotent stem cells, Biochem. Biophys. Res. Commun., 2012 Aug. 24, 425(2), 321-327).
* Human iPS cell-derived vascular endothelial cells (iPS-CD31+) were obtained by preparing with reference to the following reference (White M.P., et al.: Stem Cells, 2013 Jan., 31(1), 92-103).
* Cos-7 cells (acquired from the JCRB Cell Bank, National Institutes of Biomedical Innovation, Health and Nutrition)

### EXAMPLE 1

### Inhibition of Vascular Endothelial Network Formation by Cardiac Fibroblasts (FIG. 1)

Normal human dermal fibroblasts (NHDF) or normal human cardiac fibroblasts (atrial fibroblasts: NHCF-a, ventricular fibroblasts: NHCF-v) (2.4 × 10⁵ cells/cm²) were co-cultured with human umbilical vein endothelial cells (HUVEC) (2.0 × 10⁴ cells/cm²) for 3 days at 37°C and 5% CO₂ followed by immunostaining with anti-CD31 antibody (Human CD31/PECAM-1 PE-conjugated Antibody, FAB3567P, R & D). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

Although vascular endothelial network formation was promoted by co-culturing with normal human dermal fibroblasts, network formation was inhibited by co-culturing with normal human cardiac fibroblasts.

### EXAMPLE 2

### Inhibition of Vascular Endothelial Network Formation by Cardiac Fibroblasts (FIG. 2)

Normal human dermal fibroblasts or normal human cardiac fibroblasts (2.4 × 10⁵ cells/cm²) were co-cultured with iPS cell-derived vascular endothelial cells (iPS-CD31+) or human cardiac microvascular endothelial cells (HMVEC-C) (2.0 × 10⁴ cells/cm²) for 3 days at 37°C and 5% CO₂ followed by immunostaining with anti-CD31 antibody (Human CD31/PECAM-1 PE-conjugated Antibody, FAB3567P, R & D). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

Vascular endothelial network formation by human iPS cell-derived vascular endothelial cells and human cardiac microvascular endothelial cells was promoted by co-culturing with normal human dermal fibroblasts and was inhibited by co-culturing with normal human cardiac fibroblasts.

### EXAMPLE 3

### Inhibition of Vascular Endothelial Network Formation by Cardiac Fibroblasts (FIG. 3)

Mouse dermal fibroblasts or cardiac fibroblasts (6 × 10⁴ cells/cm²) were co-cultured with mouse ES cell-derived cardiomyocytes (2.4 × 10⁵ cells/cm²) and with mouse ES cell-derived vascular endothelial cells (2.0 × 10⁴ cells/cm²) for 3 days at 37°C and 5% CO₂ followed by immunostaining with anti-CD31 antibody (PE Rat Anti-Mouse CD31, 553373, BD Biosciences). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

Although vascular endothelial network formation by mouse ES cell-derived vascular endothelial cells was promoted in the presence of mouse dermal fibroblasts, network formation was inhibited in the presence of mouse cardiac fibroblasts.

### EXAMPLE 4

### Inhibition of Vascular Endothelial Network Formation by Cardiac Fibroblasts (FIG. 4)

Primary neonatal rat dermal fibroblasts (RDF) or rat cardiac fibroblasts (RCF) (2.4 × 10⁵ cells/cm²) collected from SD rats (Jc1:SD, Sankyo Labo Service) were co-cultured with rat neonatal cardiac vascular endothelial cells (2.0 × 10⁴ cells/cm²) for 3 days at 37°C and 5% CO₂ followed by immunostaining with anti-CD31 antibody (Mouse Anti-Rat CD31 Antibody, MCA1334G, Bio-Rad). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

Although vascular endothelial network formation was promoted by co-culturing with rat dermal fibroblasts, network formation was inhibited following co-culturing with rat cardiac fibroblasts.

### EXAMPLE 5

### Comparison of Gene Expression Levels of Dermal Fibroblasts and Cardiac Fibroblasts (FIG. 5)

Expression of genes obtained by extracting total RNA from normal human dermal fibroblasts and cardiac fibroblasts (derived from the atrium and ventricle) were analyzed with a microarray (commissioned to DNA Chip Research (Japan)). Heat maps were indicated for glycoprotein-associated genes and angiogenesis-associated genes (FIG. 5).

Gene expression patterns differed considerably between normal human dermal fibroblasts and cardiac fibroblasts. Candidate molecules were screened based on the array results and angiogenesis inhibitory factor LYPD1 was identified that is highly expressed in cardiac fibroblasts (GenBank Accession No.: NM 144586.6, SEQ ID NO: 1).

### EXAMPLE 6

### Expression of LYPD1 in Rat Cardiac Stroma (FIG. 6)

Expression of LYPD1 in various rat organs was evaluated by qPCR. Total RNA was extracted from each organ and cDNA was synthesized using mRNA contained in the total RNA fraction as template for use as the template of qPCR. qPCR was carried out by comparative CT using TaqMan® Gene Expression Assays (Rn01295701 m1, Thermo Fisher Scientific) (FIG. 6(A)). Evaluation of expression of LYPD1 in each of the rat organs revealed that LYPD1 was highly expressed in the heart.

FIG. 6(B) indicates immunostaining images of rat cardiac tissue. The tissue was stained with anti-CTnT (cardiac troponin T antibody (Anti-Troponin T, Cardiac Isoform, Mouse-Mono (13-11), AB-1, MS-295-P, Thermo Fischer Scientific), anti-LYPD1 antibody (ab157516, Abcam) and DAPI (nuclei).

When expression in rat cardiac tissue was evaluated by immunostaining, LYPD1 was not co-stained with cardiomyocytes positive for cardiac troponin T and was expressed in cardiac stroma.

### EXAMPLE 7

### Comparison of Gene Expression of LYPD1 in Human and Rat Primary Cultured Cells (FIG. 7)

Expression of LYPD1 in dermal fibroblasts and cardiac fibroblasts derived from humans and neonatal rats was evaluated by qPCR. Total RNA was extracted from each of the cells and cDNA was synthesized by using mRNA contained in the total RNA fraction as a template for use as the template of qPCR. qPCR was carried out by comparative CT using TaqMan® Gene Expression Assays (Hs00375991_m1 (human), Rn01295701_m1 (rat), Thermo Fisher Scientific).

Although hardly any LYPD1 was detected in dermal fibroblasts derived from humans and neonatal rats, LYPD1 was highly expressed in cardiac fibroblasts.

### EXAMPLE 8

### Recovery Vascular Network Formation by Inhibition of LYPD1 (FIG. 8)

After introducing siRNA to LYPD1 (Silencer® Select siRNA, Cat. No. 4392420, Thermo Fisher Scientific (1 nM) or control siRNA (Silencer® Select Negative Control No. 2 siRNA, Cat. No. 4390846 (1 nM) into human cardiac fibroblasts using Lipofectamine® RNAiMAX Transfection Reagent (Thermo Fisher Scientific) and culturing for 2 days, human cardiac fibroblasts introduced with siRNA (2.4 × 10⁵ cells/cm²) and HUVEC (2.0 × 10⁴ cells/cm²) were co-cultured for 3 days at 37°C and 5% CO₂ followed by immunostaining with anti-CD31 antibody (Human CD31/PECAM-1 PE-conjugated Antibody, FAB3567P, R & D). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

Those sequences of siRNA to LYPD1 were as indicated below.

**[Table 1]**

| SEQ ID NO: | Sequence* | Remarks |
|---|---|---|
| 17 | 5'-GGCUUUGCGCUGCAAAUCCtt-3' | Sense sequence |
| 18 | 5'-GGAUUUGCAGCGCAAAGCCtg-3' | Antisense sequence |

| | | |
|---|---|---|
| * The lower case letters on the 3'-end (tt and tg) indicate additional sequences that enhance stability. | | |

An angiogenesis inhibitory effect attributable to LYPD1 was inhibited in human cardiac fibroblasts in which expression of LYPD1 was suppressed by siRNA, and vascular network formation by co-cultured HUVEC was observed (see FIGS. 8(B) to 8(D)).

### EXAMPLE 9

### Recovery of Vascular Network Formation by Inhibition of LYPD1 (FIG. 9)

Human cardiac fibroblasts (2.4 × 10⁵ cells/cm²) and HUVEC (2.0 × 10⁴ cells/cm²) were co-cultured in the presence of anti-LYPD1 antibody (5 µg/mL) (ab157516, Abcam) or in the presence of control antibody (5 µg/mL) (normal rabbit IgG, Wako, Japan, Cat. No. 148-09551) for 4 days at 37°C and 5% CO₂ followed by immunostaining with anti-CD31 antibody (Human CD31/PECAM-1 PE-conjugated Antibody, FAB3567P, R & D) (FIGS. 9(A) and 9(B)). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC) (FIGS. 9(C) and 9(D).

An angiogenesis inhibitory effect attributable to LYPD1 expressed in human cardiac fibroblasts was inhibited in the presence of antibody to LYPD1 and vascular network formation by co-cultured HUVEC was observed.

### EXAMPLE 10

### Recovery of Vascular Network Formation by Inhibition of LYPD1 (FIG. 10)

Rat neonatal cardiac fibroblasts (2.4 × 10⁵ cells/cm²) and rat neonatal cardiac vascular endothelial cells (2.0 × 10⁴ cells/cm²) were co-cultured in the presence of anti-LYPD1 antibody (5 µg/mL) (ab157516, Abcam) or in the presence of control antibody (5 µg/mL) (normal rabbit IgG, Wako, Japan, Cat. No. 148-09551) for 4 days at 37°C and 5% CO₂ followed by immunostaining with anti-CD31 antibody (Mouse anti-Rat CD31 Antibody, MCA1334G, Bio-Rad) (FIGS. 10(A) and 10(B)). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC) (FIGS. 10(C) and 10(D)).

An angiogenesis inhibitory effect attributable to LYPD1 expressed in rat cardiac fibroblasts was inhibited in the presence of antibody to LYPD1 and vascular network formation by co-cultured rat cardiac vascular endothelial cells was observed.

### EXAMPLE 11

### Classification of iPS-Derived Stromal Cells into Clusters Identical to Cardiac Fibroblasts (FIG. 11)

Gene expression in normal human dermal fibroblasts (NHDF), normal human cardiac fibroblasts (NHCF), human iPS-derived stromal cells and human mesenchymal stem cells (Lonza, Cat. No. PT-2501) were analyzed and clustered with a microarray. The iPS-derived stromal cells were classified in the same cluster as cardiac fibroblasts.

### EXAMPLE 12

### Inhibition of Vascular Network Formation of iPS CD31-Positive Cells by iPS-Derived Stromal Cells (FIG. 12)

Human iPS-derived stromal cells and human iPS CD31-positive cells were co-cultured followed by immunostaining with anti-CD31 antibody (Human CD31/PECAM-1 PE-conjugated Antibody, FAB3567P, R & D). CD31-stained images were acquired using the ImageXpress Ultra Confocal High Content Screening System (Molecular Devices, LLC, Sunnyvale, CA, USA) (FIG. 12(B)).

Although vascular network formation of human iPS CD31-positive cells was promoted by co-culturing with human cardiac fibroblasts, network formation was inhibited during co-culturing with human iPS-derived stromal cells.

Expression of LYPD1 by normal human dermal fibroblasts (NHDF), normal human cardiac fibroblasts (NHCFa) and human iPS-derived stromal cells (iPS fibro-like) was evaluated by qPCR. Total RNA was extracted from each of the cells and cDNA was synthesized using mRNA contained in the total RNA fraction as template for use as the template of qPCR. qPCR was carried out by comparative CT using TaqMan® Gene Expression Assays (HS00375991 m1, Thermo Fisher Scientific) (FIG. 12(C)).

Expression of LYPD1 was high in human iPS-derived stromal cells in the same manner as human cardiac fibroblasts.

### EXAMPLE 13

### Expression and Purification of Recombinant LYPD1 and Confirmation of Vascular Endothelial Network Inhibitory Effect (FIG. 13)

Protein encoding the cDNA sequence of human LYPD1 was selected in accordance with published data. LYPD1 having a FLAG sequence inserted after the signal sequence was synthesized with GenScript (Piscataway, N.J., USA) and inserted into a pcDNA3.1 vector (to be referred to as pFLAG-LYPD1).

COS-7 cells were maintenance-cultured in DMEM medium supplemented with 10% fetal calf serum (Dulbecco's Modified Eagle Medium, Invitrogen) in a 5% CO₂ atmosphere at 37°C. pFLAG-LYPD1 was transfected into the COS-7 cells using Lipofectamine® 3000 (Invitrogen) in accordance with the instructions of the manufacturer. The cells were lysed with RIPA buffer (Wako, Japan) 48 hours after transfection.

FLAG-LYPD1 protein was immunoprecipitated for 3 hours at 4°C using anti-DYKDDDDK-tagged antibody beads (Wako, Japan). Then, the beads were washed three times with RIPA buffer and the FLAG-LYPD 1 protein was eluted from the beads by adding DYKDDDDK peptide (Wako, Japan). The eluate was separated in 12.5% SDS-PAGE gel and blotted on Immobilon-P (Merck, Germany).

FLAG-LYPD1 protein was detected using peroxidase-bound anti-DYKDDDDK-tagged monoclonal antibody (Wako, Japan) and rabbit polyclonal anti-LYPD1 antibody (Abcam).

Bands were visualized using the ECL Prime Western Blotting Detection Reagent (GE Healthcare UK Ltd., UK) and detected with a digital imaging system (LAS3000, GE Healthcare UK Ltd.). The amount of protein was measured with the Coomassie (Bradford) Protein Assay Kit (Thermal Scientific, Rockford, Illinois, USA) using bovine serum albumin in accordance with the instructions of the manufacturer (FIG. 13(A)).

FLAG-LYPD1 protein (1.25 µg/mL) or control IgG (1.25 µg/mL, normal rabbit IgG, Wako, Japan, Cat. No. 148-09551) was added to a cell population obtained by mixing normal human dermal fibroblasts (2.4 × 10⁵ cells/cm²) and HUVEC (2 × 10⁴ cells/cm²) followed by culturing in Dulbecco's Modified Eagle medium containing 10% fetal calf serum and 1% penicillin/streptomycin (5% CO₂, 37°C). The cells were immunostained with anti-CD31 antibody (Human CD31/PECAM-1 PE-conjugated Antibody, FAB3567P, R & D). CD31-stained images were acquired using the ImageXpress Ultra Confocal High Content Screening System (Molecular Devices, LLC, Sunnyvale, CA, USA) and regions stained with anti-CD31 antibody were taken to represent vascular endothelial cells followed by calculating the lengths and numbers of branches of vascular endothelial networks using MetaXpress software (Molecular Devices, LLC).

As a result, vascular network formation was clearly demonstrated to be inhibited by addition of recombinant LYPD1 protein (FIGS. 13(B) and 13(C)).

### EXAMPLE 14

### Recovery of Vascular Endothelial Network Formation Mediated by Suppression of LYPD1 (FIG. 14)

HUVEC (2 × 10⁴ cells/cm²) were mixed and seeded with human cardiac fibroblasts (2.4 × 10⁵ cells/cm²) transfected with LYPD1 siRNA using the same method as Example 8. Moreover, the recombinant LYPD1 (1.5 µg/mL) of Example 13 or an equal amount of buffer (composition: 500 µg/mL DYKDDDDK peptide, 10 mM Tris-HCl, pH 7.4, 150 mM NaCl) was added followed by culturing for 3 days. HUVEC (2 × 10⁴ cells/cm²) were mixed with a control in the form of normal human cardiac fibroblasts (2.4 × 10⁵ cells/cm²) transfected with control siRNA using the same method as Example 8 followed by culturing for 3 days.

Following culturing, the cells were fixed and stained with Hoechst 33342. Images were acquired using the ImageXpress Ultra Confocal High Content Screening System (Molecular Devices) and the lengths of CD31-positive cells were measured using MetaXpress software (Molecular Devices).

As a result, recovery of vascular endothelial network formation observed as a result of transfecting normal human cardiac fibroblasts with LYPD1 siRNA was re-suppressed by addition of recombinant LYPD1. This result explains that the action observed following transfection with LYPD1 siRNA is mediated by suppression of LYPD1.

### EXAMPLE 15

### Effect of LYPD1 on HUVEC Lumen Formation (FIG. 15)

46.2 µL of Matrigel® (Corning, No. 356231) were added per well (0.32 cm2) of a 96-well plate (Corning) to coat the plate. HUVEC (1 × 10⁴ cells/cm²) were suspended in 100 µL of EGM-2 (Lonza) and seeded in the Matrigel® in the presence of rLYPD1 (1µg/mL, 2 µg/mL or 5 µg/mL) or absence of rLYPD1. The plate was observed microscopically 20 hours later.

Although addition of rLYPD1 at 1 µg/mL did not have an effect on lumen formation of vascular endothelial cells, migration of vascular endothelial cells was observed to be suppressed at a concentration of 2 µg/mL, and at 5 µg/mL, lumen formation of vascular endothelial cells was completely suppressed. This result explains that the LYPD1 protein per se has actions that suppress lumen formation and migration of vascular endothelial cells.

## Claims

1. An LYPD1 inhibitor for promoting vascular endothelial network formation in biological tissue.

2. The LYPD1 inhibitor according to claim 1 for treating and/or preventing angiogenic disorders.

3. The LYPD1 inhibitor according to claim 2, wherein the angiogenic disorder is selected from the group consisting of cerebrovascular disease, cerebral infarction, transient ischemic attack, moyamoya disease, angina, (peripheral) arterial occlusion, arteriosclerosis, Buerger's disease, myocardial infarction, ischemia, cardiomyopathy, congestive heart failure, coronary artery disease, hereditary hemorrhagic telangiectasia, ischemic heart disease, vascular intimal thickening, vascular occlusion, atherosclerotic peripheral vascular disease, portal hypertension, rheumatic heart disease, hypertension, thromboembolism, atherosclerosis, post-angioplasty restenosis, pulmonary arterial hypertension, vein graft disease, hypertensive heart disease, valvular heart disease, Kawasaki disease, dilated cardiomyopathy, hypertrophic cardiomyopathy, sarcoidosis, systemic scleroderma, aortitis syndrome, asymptomatic myocardial ischemia, internal carotid artery stenosis, vertebral artery stenosis, hemodialysis cardiomyopathy, diabetic cardiomyopathy, pulmonary arterial pulmonary hypertension, ischemic cardiomyopathy, post-coronary artery bypass surgery, post-percutaneous transluminal coronary angioplasty, acute myocardial infarction, subacute myocardial infarction, old myocardial infarction, exertional angina, unstable angina, acute coronary syndrome, coronary vasospastic angina, aortic valve stenosis, aortic valve insufficiency, mitral valve insufficiency and mitral valve stenosis.

4. The LYPD1 inhibitor according to any of claims 1 to 3, wherein the biological tissue is biological tissue that expresses LYPD1.

5. The LYPD1 inhibitor according to any of claims 1 to 4, wherein the LYPD1 inhibitor is a selective LYPD1 inhibitor.

6. The LYPD1 inhibitor according to claim 5, wherein the selective LYPD1 inhibitor is selected from the group consisting of an organic small molecule, an aptamer, an antibody, an antibody fragment and a combination thereof.

7. The LYPD1 inhibitor according to any of claims 1 to 4, wherein the LYPD1 inhibitor is a LYPD1 expression inhibitor or cells treated with a LYPD1 expression inhibitor.

8. The LYPD1 inhibitor according to claim 7, the cells are provided in the form of a cell suspension or cell sheet.

9. The LYPD1 inhibitor according to claim 7 or 8, wherein the LYPD1 expression inhibitor is selected from the group consisting of an antisense RNA or DNA molecule, an RNAi-inducing nucleic acid, a microRNA (miRNA), a ribozyme, a genome-editing nucleic acid and expression vector thereof, an organic small molecule, an aptamer, an antibody, an antibody fragment and a combination thereof.

10. A pharmaceutical composition for treating and/or preventing angiogenic disorders comprising as an active ingredient thereof the LYPD1 inhibitor described in any of claims 1 to 9.

11. The pharmaceutical composition according to claim 10, further comprising one or more angiogenesis induction factors selected from the group consisting of vascular endothelial growth factor (VEGF), hepatocyte growth factor (HGF), fibroblast growth factor (FGF), epidermal growth factor (EGF), platelet-derived growth factor (PDGF), insulin-like growth factor (IGF), angiopoietin, transforming growth factor-β (TGF-β), placental growth factor (PIGF), matrix metalloproteinase (MMP), family proteins thereof and combinations thereof.

12. A method for producing biological tissue in which vascular endothelial network formation has been promoted, comprising:
(a1) a step for providing a cell population containing first cells expressing LYPD1 and vascular endothelial cells and/or vascular endothelial progenitor cells,
(a2) a step for treating the cell population obtained in step (a1) with an LYPD1 inhibitor, and
(a3) a step for culturing the cell population obtained in step (a2); or
(b1) a step for treating a cell population containing first cells expressing LYPD1 with an LYPD1 inhibitor,
(b2) a step for contacting vascular endothelial cells and/or vascular endothelial progenitor cells with the cell population obtained in step (b1), and
(b3) a step for culturing the cell population obtained in step (b2).

13. The method according to claim 12, wherein the first cells are cells derived from the heart, muscle, kidney and/or brain.

14. The method according to claim 12 or 13, wherein the LYPD1 inhibitor is selected from the group consisting of an antisense RNA or DNA molecule, an RNAi-inducing nucleic acid, a microRNA (miRNA), a ribozyme, a genome-editing nucleic acid and expression vector thereof, cells in which the expression vector has been introduced, second cells in which the expression level of LYPD1 is lower than the expression level of LYPD1 of the first cells or is not expressed at all, an organic small molecule, an aptamer, an antibody, an antibody fragment and a combination thereof.

15. The method according to claim 14, wherein the second cells are cells derived from the skin, esophagus, lung and/or liver.

16. A method for screening LYPD1 inhibitors, comprising:
(i-1) a step for providing a cell population containing first cells expressing LYPD1 and vascular endothelial cells and/or vascular endothelial progenitor cells,
(i-2) A step for treating the cell population obtained in step (i-1) with a candidate substance,
(i-3) a step for culturing the cell population obtained in step (i-2), and
(i-4) a step for evaluating formation of a vascular endothelial network in the cell population obtained in step (i-3); or,
(ii-1) a step for treating a cell population containing first cells expressing LYPD1 with a candidate substance,
(ii-2) a step for contacting vascular endothelial cells and/or vascular endothelial progenitor cells with the cell population obtained in step (ii-1),
(ii-3) a step for culturing the cell population obtained in step (ii-2), and
(ii-4) a step for evaluating formation of a vascular endothelial network in the cell population obtained in step (ii-3).


SEQUENCE LISTING

SEQUENCE LISTING

<110>  TOKYO WOMEN'S MEDICAL UNIVERSITY

<120>  LYPD1 INHIBITOR AND METHOD FOR PRODUCING BIOLOGICAL TISSUE USING
       THE SAME

<130>  P180130WO

<150>  JP 2017-042200
<151>  2017-03-06

<160>  18

<170>  PatentIn version 3.5

<210>  1
<211>  3557
<212>  DNA
<213>  Homo sapiens


<220>
<221>  CDS
<222>  (506)..(775)
<223>  Homo sapiens LY6/PLAUR domain containing 1 (LYPD1), transcript
       variant 2, mRNA

<400>  1
gaaatgaaag aagggggaga gggagggagg gagggagaga aggagcctga ggcagatatg      60

gaacgatctc tgcagaggaa ttacaaagaa agtcatttaa gcccctttac agaccagctc     120

tgctgggagg atgcagaaat aaatgatcag cgtcccggcg tgagagagct gcccccagaa     180

gtggccgggg gagaaaaggg tgaagaggag ctaaagctta ggaggcgatt tccacggaaa     240

tggatgctcc ctaaggatgt gcggaaaggg gatgggaatg tgcggattgg gggcgggggc     300

accaacgcac tccggcgggc aagcggtctc ccgcaccacc tgctgcaagg ctcgggaagg     360

aaggcgaaga tagcaccggg gcatccccgg gagtccgcaa ggctttgcgc tgcaaatcca     420

gtgctaccag tgtgaagaat tccagctgaa caacgactgc tcctcccccg agttcattgt     480

gaattgcacg gtgaacgttc aagac atg tgt cag aaa gaa gtg atg gag caa       532
                            Met Cys Gln Lys Glu Val Met Glu Gln
                            1               5

agt gcc ggg atc atg tac cgc aag tcc tgt gca tca tca gcg gcc tgt       580
Ser Ala Gly Ile Met Tyr Arg Lys Ser Cys Ala Ser Ser Ala Ala Cys
10                  15                  20                  25

ctc atc gcc tct gcc ggg tac cag tcc ttc tgc tcc cca ggg aaa ctg       628
Leu Ile Ala Ser Ala Gly Tyr Gln Ser Phe Cys Ser Pro Gly Lys Leu
                30                  35                  40

aac tca gtt tgc atc agc tgc tgc aac acc cct ctt tgt aac ggg cca       676
Asn Ser Val Cys Ile Ser Cys Cys Asn Thr Pro Leu Cys Asn Gly Pro
            45                  50                  55

agg ccc aag aaa agg gga agt tct gcc tcg gcc ctc agg cca ggg ctc       724
Arg Pro Lys Lys Arg Gly Ser Ser Ala Ser Ala Leu Arg Pro Gly Leu

60                  65                  70

cgc acc acc atc ctg ttc ctc aaa tta gcc ctc ttc tcg gca cac tgc      772
Arg Thr Thr Ile Leu Phe Leu Lys Leu Ala Leu Phe Ser Ala His Cys
    75                  80                  85

tga agctgaagga gatgccaccc cctcctgcat tgttcttcca gccctcgccc      825

ccaacccccc acctccctga gtgagtttct tctgggtgtc cttttattct gggtagggag      885

cgggagtccg tgttctcttt tgttcctgtg caaataatga aagagctcgg taaagcattc      945

tgaataaatt cagcctgact gaattttcag tatgtacttg aaggaaggag gtggagtgaa      1005

agttcacccc catgtctgtg taaccggagt caaggccagg ctggcagagt cagtccttag      1065

aagtcactga ggtgggcatc tgccttttgt aaagcctcca gtgtccattc catccctgat      1125

gggggcatag tttgagactg cagagtgaga gtgacgtttt cttagggctg gagggccagt      1185

tcccactcaa ggctccctcg cttgacattc aaacttcatg ctcctgaaaa ccattctctg      1245

cagcagaatt ggctggtttc gcgcctgagt tgggctctag tgactcgaga ctcaatgact      1305

gggacttaga ctggggctcg gcctcgctct gaaaagtgct taagaaaatc ttctcagttc      1365

tccttgcaga ggactggcgc cgggacgcga agagcaacgg gcgctgcaca aagcgggcgc      1425

tgtcggtggt ggagtgcgca tgtacgcgca ggcgcttctc gtggttggcg tgctgcagcg      1485

acaggcggca gcacagcacc tgcacgaaca cccgccgaaa ctgctgcgag gacaccgtgt      1545

acaggagcgg gttgatgacc gagctgaggt agaaaaacgt ctccgagaag gggaggagga      1605

tcatgtacgc ccggaagtag gacctcgtcc agtcgtgctt gggtttggcc gcagccatga      1665

tcctccgaat ctggttgggc atccagcata cggccaatgt cacaacaatc agccctgggc      1725

agacacgagc aggagggaga gacagagaaa agaaaaacac agcatgagaa cacagtaaat      1785

gaataaaacc ataaaatatt tagcccctct gttctgtgct tactggccag gaaatggtac      1845

caatttttca gtgttggact tgacagcttc ttttgccaca agcaagagag aatttaacac      1905

tgtttcaaac ccgggggagt tggctgtgtt aaagaaagac cattaaatgc tttagacagt      1965

gtatttatac cagttgatgt ctgttaattt taaaaaaatg ttttcattgg tgtttgtttg      2025

cgtatccaga aagcagttca tgttatccat aaatctggtt ttgtcttttt ttgttttaaa      2085

gaaaaagatg tatacataca gtatagctgc attagataaa gcagtgtttg tattttaaag      2145

gatgtctgca caaagaagac ctagtgatat ttttaaatca aatggaagaa gtgtcccttt      2205

ggcaacaaag cagcatattt aatgacactg gttttgcatt cagtttcagg ggaagcaaag      2265

tcaggaatag cctgtcgcca agaatgtttt ttggacatat acatactagg tatgcacacc      2325

tataatcatg atgctcatat ctgcaacagc atatgtgttt cttttcagac acttttagat      2385

ccctcatgtg gggaaaaaga attattcaga gatggcaaat ataaaacttc cttctagttc      2445

agccagtaac atgttccctt cctttgcagc actgagctgt gctgtcaaca gcccagaagc      2505

aatcaggccc tagagaagag accactcaaa ggcccttctg tagatcaaat gtttactgca 2565

tgtacatttg tttgcatgcc cacatatttg tattccaact taagtaacca ccaccagttc 2625

tgcaattctg actgacagag ataaagatgc tacatagacc acaaacaact gaaatcacag 2685

gtatcatgag agtttagtta cagtgacaaa agcaaaaaag aacaaaggaa gatcagggga 2745

tctgtgaagc atttgctctc tcttttcgta aggagccaag acacccacag taaattcccc 2805

tgtagagagc tgctacctta aagcaggatt tgcattttca gaaatgcttc cttcctctcc 2865

tacatttcaa tcgtagtaag aaacatttac tcacattttc aatcttctga ttttctagaa 2925

accctaggga agtgacagtt ggcaatgaat gcttcctgcc tatgacccat ggtaaatatt 2985

ctattaataa atgggggcca gacatggtgg cgcatgcctg atatctcaat actctgggag 3045

gccaaggcag aaggatcact taagcctaga aatttgagac ccacctaggc aacatagcaa 3105

gaccccatct ctacaaaaaa agaaaaactt agccaggcat ggtggtacat acacacctgt 3165

ggtctcagat actttttggg ggctgaggcg ggaggatcac ttgagcccag gaggtgaagg 3225

ctacagtgag acacgaatgt gccactgcac tccagcctgg ctgacagagt gaaactgtct 3285

caataaacca ataaataaat gctccaggaa aaaacagcca cattcacaca tccagaattg 3345

agcctcctgt atgcactggc ctgagtattc cttgcctgct gttggagggg accctagctg 3405

tgttcaaatc ctccacaaat ccatatgtga gcaaggaagg ccttggaaac tcttctcctt 3465

tgttaatttc cacaggtttc tcctgtcaac tcccagccta aaactttgaa atataagcca 3525

atttgtttat tttttaaaaa aaaaaaaaaa aa 3557

<210> 2
<211> 89
<212> PRT
<213> Homo sapiens

<400> 2

Met Cys Gln Lys Glu Val Met Glu Gln Ser Ala Gly Ile Met Tyr Arg
1               5                   10                  15

Lys Ser Cys Ala Ser Ser Ala Ala Cys Leu Ile Ala Ser Ala Gly Tyr
            20                  25                  30

Gln Ser Phe Cys Ser Pro Gly Lys Leu Asn Ser Val Cys Ile Ser Cys
        35                  40                  45

Cys Asn Thr Pro Leu Cys Asn Gly Pro Arg Pro Lys Lys Arg Gly Ser
    50                  55                  60

Ser Ala Ser Ala Leu Arg Pro Gly Leu Arg Thr Thr Ile Leu Phe Leu
65                  70                  75                  80

Lys Leu Ala Leu Phe Ser Ala His Cys
                85

<210>  3
<211>  3646
<212>  DNA
<213>  Homo sapiens

<220>
<221>  CDS
<222>  (439)..(864)
<223>  Homo sapiens LY6/PLAUR domain containing 1 (LYPD1), transcript
       variant 1, mRNA

<400>  3
gcgggagaag aggaagacag gaagggggtg gggatgtgaa gcgaccgtcc cagccttccc      60

cgcccgccac ccccacccca actcggcagc cgtcacgtga tgcctggagt gggaggtggg     120

gagaaaaggc gagacttttg tgggtgctcc cgatcgccag tagttccttc agtctcagcc     180

gccaactccg gaggcgcggt gctcggcccg ggagcgcgag cgggaggagc agagacccgc     240

agccgggagc ccgagcgcgg gcgatgcagg ctccgcgagc ggcacctgcg gctcctctaa     300

gctacgaccg tcgtctccgc ggcagcagcg cgggccccag cagcctcggc agccacagcc     360

gctgcagccg gggcagcctc cgctgctgtc gcctcctctg atgcgcttgc cctctcccgg     420

ccccgggact ccgggaga atg tgg gtc cta ggc atc gcg gca act ttt tgc       471
                    Met Trp Val Leu Gly Ile Ala Ala Thr Phe Cys
                    1               5                   10

gga ttg ttc ttg ctt cca ggc ttt gcg ctg caa atc cag tgc tac cag       519
Gly Leu Phe Leu Leu Pro Gly Phe Ala Leu Gln Ile Gln Cys Tyr Gln
            15                  20                  25

tgt gaa gaa ttc cag ctg aac aac gac tgc tcc tcc ccc gag ttc att       567
Cys Glu Glu Phe Gln Leu Asn Asn Asp Cys Ser Ser Pro Glu Phe Ile
        30                  35                  40

gtg aat tgc acg gtg aac gtt caa gac atg tgt cag aaa gaa gtg atg       615
Val Asn Cys Thr Val Asn Val Gln Asp Met Cys Gln Lys Glu Val Met
    45                  50                  55

gag caa agt gcc ggg atc atg tac cgc aag tcc tgt gca tca tca gcg       663
Glu Gln Ser Ala Gly Ile Met Tyr Arg Lys Ser Cys Ala Ser Ser Ala
60                  65                  70                  75

gcc tgt ctc atc gcc tct gcc ggg tac cag tcc ttc tgc tcc cca ggg       711
Ala Cys Leu Ile Ala Ser Ala Gly Tyr Gln Ser Phe Cys Ser Pro Gly
                80                  85                  90

aaa ctg aac tca gtt tgc atc agc tgc tgc aac acc cct ctt tgt aac       759
Lys Leu Asn Ser Val Cys Ile Ser Cys Cys Asn Thr Pro Leu Cys Asn
            95                  100                 105

ggg cca agg ccc aag aaa agg gga agt tct gcc tcg gcc ctc agg cca       807
Gly Pro Arg Pro Lys Lys Arg Gly Ser Ser Ala Ser Ala Leu Arg Pro

        110                 115                 120

ggg ctc cgc acc acc atc ctg ttc ctc aaa tta gcc ctc ttc tcg gca 855
Gly Leu Arg Thr Thr Ile Leu Phe Leu Lys Leu Ala Leu Phe Ser Ala
    125                 130                 135

cac tgc tga agctgaagga gatgccaccc cctcctgcat tgttcttcca 904
His Cys
140

gccctcgccc ccaacccccc acctccctga gtgagtttct tctgggtgtc cttttattct 964

gggtagggag cgggagtccg tgttctcttt tgttcctgtg caaataatga aagagctcgg 1024

taaagcattc tgaataaatt cagcctgact gaattttcag tatgtacttg aaggaaggag 1084

gtggagtgaa agttcacccc catgtctgtg taaccggagt caaggccagg ctggcagagt 1144

cagtccttag aagtcactga ggtgggcatc tgccttttgt aaagcctcca gtgtccattc 1204

catccctgat gggggcatag tttgagactg cagagtgaga gtgacgtttt cttagggctg 1264

gagggccagt tcccactcaa ggctccctcg cttgacattc aaacttcatg ctcctgaaaa 1324

ccattctctg cagcagaatt ggctggtttc gcgcctgagt tgggctctag tgactcgaga 1384

ctcaatgact gggacttaga ctggggctcg gcctcgctct gaaaagtgct taagaaaatc 1444

ttctcagttc tccttgcaga ggactggcgc cgggacgcga agagcaacgg gcgctgcaca 1504

aagcgggcgc tgtcggtggt ggagtgcgca tgtacgcgca ggcgcttctc gtggttggcg 1564

tgctgcagcg acaggcggca gcacagcacc tgcacgaaca cccgccgaaa ctgctgcgag 1624

gacaccgtgt acaggagcgg gttgatgacc gagctgaggt agaaaaacgt ctccgagaag 1684

gggaggagga tcatgtacgc ccggaagtag gacctcgtcc agtcgtgctt gggtttggcc 1744

gcagccatga tcctccgaat ctggttgggc atccagcata cggccaatgt cacaacaatc 1804

agccctgggc agacacgagc aggagggaga gacagagaaa agaaaaacac agcatgagaa 1864

cacagtaaat gaataaaacc ataaaatatt tagcccctct gttctgtgct tactggccag 1924

gaaatggtac caatttttca gtgttggact tgacagcttc ttttgccaca agcaagagag 1984

aatttaacac tgtttcaaac ccgggggagt tggctgtgtt aaagaaagac cattaaatgc 2044

tttagacagt gtatttatac cagttgatgt ctgttaattt taaaaaaatg ttttcattgg 2104

tgtttgtttg cgtatccaga aagcagttca tgttatccat aaatctggtt ttgtcttttt 2164

ttgttttaaa gaaaaagatg tatacataca gtatagctgc attagataaa gcagtgtttg 2224

tattttaaag gatgtctgca caaagaagac ctagtgatat ttttaaatca aatggaagaa 2284

gtgtcccttt ggcaacaaag cagcatattt aatgacactg gttttgcatt cagtttcagg 2344

ggaagcaaag tcaggaatag cctgtcgcca agaatgtttt ttggacatat acatactagg 2404

tatgcacacc tataatcatg atgctcatat ctgcaacagc atatgtgttt cttttcagac 2464

acttttagat ccctcatgtg ggaaaaaaga attattcaga gatggcaaat ataaaacttc 2524

cttctagttc agccagtaac atgttccctt cctttgcagc actgagctgt gctgtcaaca 2584

gcccagaagc aatcaggccc tagagaagag accactcaaa ggcccttctg tagatcaaat 2644

gtttactgca tgtacatttg tttgcatgcc cacatatttg tattccaact taagtaacca 2704

ccaccagttc tgcaattctg actgacagag ataaagatgc tacatagacc acaaacaact 2764

gaaatcacag gtatcatgag agtttagtta cagtgacaaa agcaaaaaag aacaaaggaa 2824

gatcagggga tctgtgaagc atttgctctc tcttttcgta aggagccaag acacccacag 2884

taaattcccc tgtagagagc tgctacctta aagcaggatt tgcattttca gaaatgcttc 2944

cttcctctcc tacatttcaa tcgtagtaag aaacatttac tcacattttc aatcttctga 3004

ttttctagaa accctaggga agtgacagtt ggcaatgaat gcttcctgcc tatgacccat 3064

ggtaaatatt ctattaataa atgggggcca gacatggtgg cgcatgcctg atatctcaat 3124

actctgggag gccaaggcag aaggatcact taagcctaga aatttgagac ccacctaggc 3184

aacatagcaa gaccccatct ctacaaaaaa agaaaaactt agccaggcat ggtggtacat 3244

acacacctgt ggtctcagat acttttgggg ggctgaggcg ggaggatcac ttgagcccag 3304

gaggtgaagg ctacagtgag acacgaatgt gccactgcac tccagcctgg ctgacagagt 3364

gaaactgtct caataaacca ataaataaat gctccaggaa aaaacagcca cattcacaca 3424

tccagaattg agcctcctgt atgcactggc ctgagtattc cttgcctgct gttggagggg 3484

accctagctg tgttcaaatc ctccacaaat ccatatgtga gcaaggaagg ccttggaaac 3544

tcttctcctt tgttaatttc cacaggtttc tcctgtcaac tcccagccta aaactttgaa 3604

atataagcca atttgtttat tttttaaaaa aaaaaaaaaa aa 3646

<210> 4
<211> 141
<212> PRT
<213> Homo sapiens

<400> 4

Met Trp Val Leu Gly Ile Ala Ala Thr Phe Cys Gly Leu Phe Leu Leu
1               5                   10                  15

Pro Gly Phe Ala Leu Gln Ile Gln Cys Tyr Gln Cys Glu Glu Phe Gln
            20                  25                  30

Leu Asn Asn Asp Cys Ser Ser Pro Glu Phe Ile Val Asn Cys Thr Val
        35                  40                  45

Asn Val Gln Asp Met Cys Gln Lys Glu Val Met Glu Gln Ser Ala Gly
    50                  55                  60

Ile Met Tyr Arg Lys Ser Cys Ala Ser Ser Ala Ala Cys Leu Ile Ala
65                  70                  75                  80

Ser Ala Gly Tyr Gln Ser Phe Cys Ser Pro Gly Lys Leu Asn Ser Val
                85                  90                  95

Cys Ile Ser Cys Cys Asn Thr Pro Leu Cys Asn Gly Pro Arg Pro Lys
            100                 105                 110

Lys Arg Gly Ser Ser Ala Ser Ala Leu Arg Pro Gly Leu Arg Thr Thr
        115                 120                 125

Ile Leu Phe Leu Lys Leu Ala Leu Phe Ser Ala His Cys
    130                 135                 140

<210>  5
<211>  3519
<212>  DNA
<213>  Homo sapiens

<220>
<221>  CDS
<222>  (264)..(737)
<223>  Homo sapiens LY6/PLAUR domain containing 1 (LYPD1), transcript
       variant 3, mRNA

<400>  5
gcgggagaag aggaagacag gaagggggtg gggatgtgaa gcgaccgtcc cagccttccc      60

cgcccgccac ccccacccca actcggcagc cgtcacgtga tgcctggagt gggaggtggg     120

gagaaaaggc gagacttttg tgggtgctcc cgatcgccag tagttccttc agtctcagcc     180

gccaactccg gaggcgcggt gctcggcccg ggagcgcgag cgggaggagc agagacccgc     240

agccgggagc ccgagcgcgg gcg atg cag gct ccg cga gcg gca cct gcg gct     293
                          Met Gln Ala Pro Arg Ala Ala Pro Ala Ala
                          1               5                   10

cct cta agc tac gac cgt cgt ctc cgc ggc agc atc gcg gca act ttt       341
Pro Leu Ser Tyr Asp Arg Arg Leu Arg Gly Ser Ile Ala Ala Thr Phe
                15                  20                  25

tgc gga ttg ttc ttg ctt cca ggc ttt gcg ctg caa atc cag tgc tac       389
Cys Gly Leu Phe Leu Leu Pro Gly Phe Ala Leu Gln Ile Gln Cys Tyr
            30                  35                  40

cag tgt gaa gaa ttc cag ctg aac aac gac tgc tcc tcc ccc gag ttc       437
Gln Cys Glu Glu Phe Gln Leu Asn Asn Asp Cys Ser Ser Pro Glu Phe
        45                  50                  55

att gtg aat tgc acg gtg aac gtt caa gac atg tgt cag aaa gaa gtg       485
Ile Val Asn Cys Thr Val Asn Val Gln Asp Met Cys Gln Lys Glu Val
    60                  65                  70

atg gag caa agt gcc ggg atc atg tac cgc aag tcc tgt gca tca tca       533
Met Glu Gln Ser Ala Gly Ile Met Tyr Arg Lys Ser Cys Ala Ser Ser

75                  80                  85                  90

gcg gcc tgt ctc atc gcc tct gcc ggg tac cag tcc ttc tgc tcc cca      581
Ala Ala Cys Leu Ile Ala Ser Ala Gly Tyr Gln Ser Phe Cys Ser Pro
                95                  100                 105

ggg aaa ctg aac tca gtt tgc atc agc tgc tgc aac acc cct ctt tgt      629
Gly Lys Leu Asn Ser Val Cys Ile Ser Cys Cys Asn Thr Pro Leu Cys
            110                 115                 120

aac ggg cca agg ccc aag aaa agg gga agt tct gcc tcg gcc ctc agg      677
Asn Gly Pro Arg Pro Lys Lys Arg Gly Ser Ser Ala Ser Ala Leu Arg
        125                 130                 135

cca ggg ctc cgc acc acc atc ctg ttc ctc aaa tta gcc ctc ttc tcg      725
Pro Gly Leu Arg Thr Thr Ile Leu Phe Leu Lys Leu Ala Leu Phe Ser
    140                 145                 150

gca cac tgc tga agctgaagga gatgccaccc cctcctgcat tgttcttcca          777
Ala His Cys
155

gccctcgccc ccaacccccc acctccctga gtgagtttct tctgggtgtc cttttattct      837

gggtagggag cgggagtccg tgttctcttt tgttcctgtg caaataatga aagagctcgg      897

taaagcattc tgaataaatt cagcctgact gaattttcag tatgtacttg aaggaaggag      957

gtggagtgaa agttcacccc catgtctgtg taaccggagt caaggccagg ctggcagagt     1017

cagtccttag aagtcactga ggtgggcatc tgccttttgt aaagcctcca gtgtccattc     1077

catccctgat gggggcatag tttgagactg cagagtgaga gtgacgtttt cttagggctg     1137

gagggccagt tcccactcaa ggctccctcg cttgacattc aaacttcatg ctcctgaaaa     1197

ccattctctg cagcagaatt ggctggtttc gcgcctgagt tgggctctag tgactcgaga     1257

ctcaatgact gggacttaga ctggggctcg gcctcgctct gaaaagtgct taagaaaatc     1317

ttctcagttc tccttgcaga ggactggcgc cgggacgcga agagcaacgg gcgctgcaca     1377

aagcgggcgc tgtcggtggt ggagtgcgca tgtacgcgca ggcgcttctc gtggttggcg     1437

tgctgcagcg acaggcggca gcacagcacc tgcacgaaca cccgccgaaa ctgctgcgag     1497

gacaccgtgt acaggagcgg gttgatgacc gagctgaggt agaaaaacgt ctccgagaag     1557

gggaggagga tcatgtacgc ccggaagtag gacctcgtcc agtcgtgctt gggtttggcc     1617

gcagccatga tcctccgaat ctggttgggc atccagcata cggccaatgt cacaacaatc     1677

agccctgggc agacacgagc aggagggaga gacagagaaa agaaaaacac agcatgagaa     1737

cacagtaaat gaataaaacc ataaaatatt tagcccctct gttctgtgct tactggccag     1797

gaaatggtac caatttttca gtgttggact tgacagcttc ttttgccaca agcaagagag     1857

aatttaacac tgtttcaaac ccgggggagt tggctgtgtt aaagaaagac cattaaatgc     1917

tttagacagt gtatttatac cagttgatgt ctgttaattt taaaaaaatg ttttcattgg     1977

tgtttgtttg cgtatccaga aagcagttca tgttatccat aaatctggtt ttgtcttttt     2037

ttgttttaaa gaaaaagatg tatacataca gtatagctgc attagataaa gcagtgtttg 2097

tattttaaag gatgtctgca caaagaagac ctagtgatat ttttaaatca aatggaagaa 2157

gtgtcccttt ggcaacaaag cagcatattt aatgacactg gttttgcatt cagtttcagg 2217

ggaagcaaag tcaggaatag cctgtcgcca agaatgtttt ttggacatat acatactagg 2277

tatgcacacc tataatcatg atgctcatat ctgcaacagc atatgtgttt cttttcagac 2337

acttttagat ccctcatgtg gggaaaaaga attattcaga gatggcaaat ataaaacttc 2397

cttctagttc agccagtaac atgttccctt cctttgcagc actgagctgt gctgtcaaca 2457

gcccagaagc aatcaggccc tagagaagag accactcaaa ggcccttctg tagatcaaat 2517

gtttactgca tgtacatttg tttgcatgcc cacatatttg tattccaact taagtaacca 2577

ccaccagttc tgcaattctg actgacagag ataaagatgc tacatagacc acaaacaact 2637

gaaatcacag gtatcatgag agtttagtta cagtgacaaa agcaaaaaag aacaaaggaa 2697

gatcagggga tctgtgaagc atttgctctc tcttttcgta aggagccaag acacccacag 2757

taaattcccc tgtagagagc tgctacctta aagcaggatt tgcattttca gaaatgcttc 2817

cttcctctcc tacatttcaa tcgtagtaag aaacatttac tcacattttc aatcttctga 2877

ttttctagaa accctaggga agtgacagtt ggcaatgaat gcttcctgcc tatgacccat 2937

ggtaaatatt ctattaataa atgggggcca gacatggtgg cgcatgcctg atatctcaat 2997

actctgggag gccaaggcag aaggatcact taagcctaga aatttgagac ccacctaggc 3057

aacatagcaa gaccccatct ctacaaaaaa agaaaaactt agccaggcat ggtggtacat 3117

acacacctgt ggtctcagat acttttgggg ggctgaggcg ggaggatcac ttgagcccag 3177

gaggtgaagg ctacagtgag acacgaatgt gccactgcac tccagcctgg ctgacagagt 3237

gaaactgtct caataaacca ataaataaat gctccaggaa aaaacagcca cattcacaca 3297

tccagaattg agcctcctgt atgcactggc ctgagtattc cttgcctgct gttggagggg 3357

accctagctg tgttcaaatc ctccacaaat ccatatgtga gcaaggaagg ccttggaaac 3417

tcttctcctt tgttaatttc cacaggtttc tcctgtcaac tcccagccta aaactttgaa 3477

atataagcca atttgtttat tttttaaaaa aaaaaaaaaa aa 3519

<210> 6
<211> 157
<212> PRT
<213> Homo sapiens

<400> 6

Met Gln Ala Pro Arg Ala Ala Pro Ala Ala Pro Leu Ser Tyr Asp Arg
1               5                   10                  15

Arg Leu Arg Gly Ser Ile Ala Ala Thr Phe Cys Gly Leu Phe Leu Leu
            20                  25                  30

Pro Gly Phe Ala Leu Gln Ile Gln Cys Tyr Gln Cys Glu Glu Phe Gln
        35                  40                  45

Leu Asn Asn Asp Cys Ser Ser Pro Glu Phe Ile Val Asn Cys Thr Val
    50                  55                  60

Asn Val Gln Asp Met Cys Gln Lys Glu Val Met Glu Gln Ser Ala Gly
65                  70                  75                  80

Ile Met Tyr Arg Lys Ser Cys Ala Ser Ser Ala Ala Cys Leu Ile Ala
                85                  90                  95

Ser Ala Gly Tyr Gln Ser Phe Cys Ser Pro Gly Lys Leu Asn Ser Val
            100                 105                 110

Cys Ile Ser Cys Cys Asn Thr Pro Leu Cys Asn Gly Pro Arg Pro Lys
        115                 120                 125

Lys Arg Gly Ser Ser Ala Ser Ala Leu Arg Pro Gly Leu Arg Thr Thr
    130                 135                 140

Ile Leu Phe Leu Lys Leu Ala Leu Phe Ser Ala His Cys
145                 150                 155

<210>  7
<211>  3680
<212>  DNA
<213>  Homo sapiens

<220>
<221>  CDS
<222>  (629)..(898)
<223>  Homo sapiens LY6/PLAUR domain containing 1 (LYPD1), transcript
       variant 4, mRNA

<400>  7
gcgggagaag aggaagacag gaagggggtg gggatgtgaa gcgaccgtcc cagccttccc      60

cgcccgccac ccccacccca actcggcagc cgtcacgtga tgcctggagt gggaggtggg     120

gagaaaaggc gagacttttg tgggtgctcc cgatcgccag tagttccttc agtctcagcc     180

gccaactccg gaggcgcggt gctcggcccg ggagcgcgag cgggaggagc agagacccgc     240

agccgggagc ccgagcgcgg gcgatgcagg ctccgcgagc ggcacctgcg gctcctctaa     300

gctacgaccg tcgtctccgc ggcagcagcg cgggccccag cagcctcggc agccacagcc     360

gctgcagccg gggcagcctc cgctgctgtc gcctcctctg atgcgcttgc cctctcccgg     420

ccccgggact ccgggagaat gtgggtccta ggcatcgcgg caactttttg cggattgttc 480

ttgcttccag gtgagaatac ccagaggcca gcagccgagg ccaggctttg cgctgcaaat 540

ccagtgctac cagtgtgaag aattccagct gaacaacgac tgctcctccc ccgagttcat 600

tgtgaattgc acggtgaacg ttcaagac atg tgt cag aaa gaa gtg atg gag 652
Met Cys Gln Lys Glu Val Met Glu
1               5

caa agt gcc ggg atc atg tac cgc aag tcc tgt gca tca tca gcg gcc 700
Gln Ser Ala Gly Ile Met Tyr Arg Lys Ser Cys Ala Ser Ser Ala Ala
    10                  15                  20

tgt ctc atc gcc tct gcc ggg tac cag tcc ttc tgc tcc cca ggg aaa 748
Cys Leu Ile Ala Ser Ala Gly Tyr Gln Ser Phe Cys Ser Pro Gly Lys
25                  30                  35                  40

ctg aac tca gtt tgc atc agc tgc tgc aac acc cct ctt tgt aac ggg 796
Leu Asn Ser Val Cys Ile Ser Cys Cys Asn Thr Pro Leu Cys Asn Gly
                45                  50                  55

cca agg ccc aag aaa agg gga agt tct gcc tcg gcc ctc agg cca ggg 844
Pro Arg Pro Lys Lys Arg Gly Ser Ser Ala Ser Ala Leu Arg Pro Gly
            60                  65                  70

ctc cgc acc acc atc ctg ttc ctc aaa tta gcc ctc ttc tcg gca cac 892
Leu Arg Thr Thr Ile Leu Phe Leu Lys Leu Ala Leu Phe Ser Ala His
        75                  80                  85

tgc tga agctgaagga gatgccaccc cctcctgcat tgttcttcca gccctcgccc 948
Cys

ccaacccccc acctccctga gtgagtttct tctgggtgtc cttttattct gggtagggag 1008

cgggagtccg tgttctcttt tgttcctgtg caaataatga aagagctcgg taaagcattc 1068

tgaataaatt cagcctgact gaattttcag tatgtacttg aaggaaggag gtggagtgaa 1128

agttcacccc catgtctgtg taaccggagt caaggccagg ctggcagagt cagtccttag 1188

aagtcactga ggtgggcatc tgccttttgt aaagcctcca gtgtccattc catccctgat 1248

gggggcatag tttgagactg cagagtgaga gtgacgtttt cttagggctg gagggccagt 1308

tcccactcaa ggctccctcg cttgacattc aaacttcatg ctcctgaaaa ccattctctg 1368

cagcagaatt ggctggtttc gcgcctgagt tgggctctag tgactcgaga ctcaatgact 1428

gggacttaga ctggggctcg gcctcgctct gaaaagtgct taagaaaatc ttctcagttc 1488

tccttgcaga ggactggcgc cgggacgcga agagcaacgg gcgctgcaca aagcgggcgc 1548

tgtcggtggt ggagtgcgca tgtacgcgca ggcgcttctc gtggttggcg tgctgcagcg 1608

acaggcggca gcacagcacc tgcacgaaca cccgccgaaa ctgctgcgag gacaccgtgt 1668

acaggagcgg gttgatgacc gagctgaggt agaaaaacgt ctccgagaag gggaggagga 1728

tcatgtacgc ccggaagtag gacctcgtcc agtcgtgctt gggtttggcc gcagccatga 1788

tcctccgaat ctggttgggc atccagcata cggccaatgt cacaacaatc agccctgggc 1848

agacacgagc aggagggaga gacagagaaa agaaaaacac agcatgagaa cacagtaaat 1908

gaataaaacc ataaaatatt tagcccctct gttctgtgct tactggccag gaaatggtac 1968

caatttttca gtgttggact tgacagcttc ttttgccaca agcaagagag aatttaacac 2028

tgtttcaaac ccgggggagt tggctgtgtt aaagaaagac cattaaatgc tttagacagt 2088

gtatttatac cagttgatgt ctgttaattt taaaaaaatg ttttcattgg tgtttgtttg 2148

cgtatccaga aagcagttca tgttatccat aaatctggtt ttgtcttttt ttgttttaaa 2208

gaaaaagatg tatacataca gtatagctgc attagataaa gcagtgtttg tattttaaag 2268

gatgtctgca caaagaagac ctagtgatat ttttaaatca aatggaagaa gtgtcccttt 2328

ggcaacaaag cagcatattt aatgacactg gttttgcatt cagtttcagg ggaagcaaag 2388

tcaggaatag cctgtcgcca agaatgtttt ttggacatat acatactagg tatgcacacc 2448

tataatcatg atgctcatat ctgcaacagc atatgtgttt cttttcagac acttttagat 2508

ccctcatgtg ggaaaaaaga attattcaga gatggcaaat ataaaacttc cttctagttc 2568

agccagtaac atgttccctt cctttgcagc actgagctgt gctgtcaaca gcccagaagc 2628

aatcaggccc tagagaagag accactcaaa ggcccttctg tagatcaaat gtttactgca 2688

tgtacatttg tttgcatgcc cacatatttg tattccaact taagtaacca ccaccagttc 2748

tgcaattctg actgacagag ataaagatgc tacatagacc acaaacaact gaaatcacag 2808

gtatcatgag agtttagtta cagtgacaaa agcaaaaaag aacaaaggaa gatcagggga 2868

tctgtgaagc atttgctctc tcttttcgta aggagccaag acacccacag taaattcccc 2928

tgtagagagc tgctacctta aagcaggatt tgcattttca gaaatgcttc cttcctctcc 2988

tacatttcaa tcgtagtaag aaacatttac tcacattttc aatcttctga ttttctagaa 3048

accctaggga agtgacagtt ggcaatgaat gcttcctgcc tatgacccat ggtaaatatt 3108

ctattaataa atgggggcca gacatggtgg cgcatgcctg atatctcaat actctgggag 3168

gccaaggcag aaggatcact taagcctaga aatttgagac ccacctaggc aacatagcaa 3228

gaccccatct ctacaaaaaa agaaaaactt agccaggcat ggtggtacat acacacctgt 3288

ggtctcagat acttttgggg ggctgaggcg ggaggatcac ttgagcccag gaggtgaagg 3348

ctacagtgag acacgaatgt gccactgcac tccagcctgg ctgacagagt gaaactgtct 3408

caataaacca ataaataaat gctccaggaa aaaacagcca cattcacaca tccagaattg 3468

agcctcctgt atgcactggc ctgagtattc cttgcctgct gttggagggg accctagctg 3528

tgttcaaatc ctccacaaat ccatatgtga gcaaggaagg ccttggaaac tcttctcctt 3588

tgttaatttc cacaggtttc tcctgtcaac tcccagccta aaactttgaa atataagcca 3648

atttgtttat tttttaaaaa aaaaaaaaaa aa 3680

<210>  8
<211>  89
<212>  PRT
<213>  Homo sapiens

<400>  8

Met Cys Gln Lys Glu Val Met Glu Gln Ser Ala Gly Ile Met Tyr Arg
1               5                   10                  15

Lys Ser Cys Ala Ser Ser Ala Ala Cys Leu Ile Ala Ser Ala Gly Tyr
            20                  25                  30

Gln Ser Phe Cys Ser Pro Gly Lys Leu Asn Ser Val Cys Ile Ser Cys
        35                  40                  45

Cys Asn Thr Pro Leu Cys Asn Gly Pro Arg Pro Lys Lys Arg Gly Ser
    50                  55                  60

Ser Ala Ser Ala Leu Arg Pro Gly Leu Arg Thr Thr Ile Leu Phe Leu
65                  70                  75                  80

Lys Leu Ala Leu Phe Ser Ala His Cys
                85

<210>  9
<211>  6480
<212>  DNA
<213>  Mus musculus

<220>
<221>  CDS
<222>  (325)..(750)
<223>  Mus musculus Ly6/Plaur domain containing 1 (Lypd1), transcript
       variant 1, mRNA

<400>  9
agaagaggcg agactttttt gggtgctccg gatcgccagt agttcttcaa gcctcagcag      60

ccaactcctc cggaggcgct gcgctccgcc ccagggagcg cgaatccaag gagcctggga     120

ccagcctctg ggagcccccg gcgcgggcga tgcgggcgcc gcgggcgaca cctgcggctc     180

ctctcggtgg cagccgtcgc ttgggcggca gcagcgcgag cctcggcagc ctcggcagct     240

actgtcgccg cggccagaac agcctccgct gcggtcgtgg tctctgatgc tcttgcccgc     300

tcccggccct gccgatccgg gagg atg tgg gtt ctc ggc atc gca gca act       351
                           Met Trp Val Leu Gly Ile Ala Ala Thr
                           1               5

ttt tgc gga ttg ttc tgg ctt cca ggg ctg gcg ctg caa att cag tgc      399
Phe Cys Gly Leu Phe Trp Leu Pro Gly Leu Ala Leu Gln Ile Gln Cys
10                  15                  20                  25

tac cag tgt gaa gaa ttc cag ctg aac aac gat tgc tca tcc cct gag     447
Tyr Gln Cys Glu Glu Phe Gln Leu Asn Asn Asp Cys Ser Ser Pro Glu
                30                  35                  40

ttc atc gta aat tgc acc gtg aac gtt caa gac atg tgt cag aaa gaa     495
Phe Ile Val Asn Cys Thr Val Asn Val Gln Asp Met Cys Gln Lys Glu
            45                  50                  55

gtg atg gag caa agt gct ggg atc atg tac cgg aag tcg tgt gca tcg     543
Val Met Glu Gln Ser Ala Gly Ile Met Tyr Arg Lys Ser Cys Ala Ser
        60                  65                  70

tca gca gcc tgt ctc att gct tca gct ggg tac cag tcc ttc tgt tcc     591
Ser Ala Ala Cys Leu Ile Ala Ser Ala Gly Tyr Gln Ser Phe Cys Ser
    75                  80                  85

cct ggg aaa ctg aac tcc gtg tgc atc agc tgc tgc aac acc cct ctt     639
Pro Gly Lys Leu Asn Ser Val Cys Ile Ser Cys Cys Asn Thr Pro Leu
90                  95                  100                 105

tgc aat ggg ccg agg ccc aag aag aga ggc agc tct gcc tcg gcc atc     687
Cys Asn Gly Pro Arg Pro Lys Lys Arg Gly Ser Ser Ala Ser Ala Ile
                110                 115                 120

agg cca ggg ctt ctc acc act ctc ctg ttc ttc cac tta gcc ctc tgc     735
Arg Pro Gly Leu Leu Thr Thr Leu Leu Phe Phe His Leu Ala Leu Cys
            125                 130                 135

ttg gca cac tgc tga agctaaagga gatgccaacc cctgctgcct cacctgtctg     790
Leu Ala His Cys
        140

gcccttcgtc tctcaccttc ccgagtctct tctgggtgtc cttttattct gggtagacaa     850

gggagtcttt ttgttccctc ctttcaagta acgcaagatt gccgtgcaca aatacttttg     910

taagctctga accaattcat tctgaatttc tgtgtgtagt tgaagaaaaa agcatggagc     970

agaaagtcca gaccctccca tcccaatctg gttaaccacc gccaaggcta gcctggaaga    1030

accagccctt agaagtcatt gagatacgca tctgcctttc ccaaagcctt gagcttccat    1090

tctgtcccag taggagtcac agtctattca gagactgctg ctgcgtgaag gtaactttgc    1150

ttttgcggga ggggagagcc agtttcggct caaggcttct gaacttgcca ttcatacttc    1210

ctgctcctgt aaactatttt ctggggtgga cccagctggt ttggtctctg agccagtctg    1270

tggtgactca ggactcaagg gctggggctt agcctctcca ggcttggcct cagtctgaaa    1330

agtgcttaag aaaaccttgt tagttctcct ggaggaagag ttactgcgcc gggaggctag    1390

gaagatgagg gggctgcggg ctgagctggt gctgtccttg gtggagatga agcgggcacg    1450

ctggcgtttc tcttggttgg catgctgcag agtcaggcgg cagcagagca cctgccagaa    1510

caccttccgg aactgctgag aggacacgtt gtagaggaga gggttgacca cagagctgag    1570

gtagaagaag gtatcagaga agggcaggag gatcatgtat gccctgaagt acgttctggt    1630

ccagtcatgt ttgggttttg ctgcagccat gatccgtcgg atctgattgg gcatccaaca    1690

cacggccaac gtcaccacaa tcagtcctgg caggcaagaa caggagagaa aaggagacgg    1750

ggagagaaac agcatgagaa caaaaataaa taaataaaaa cccataaaat attaagcccc 1810

ttggttctgt tgcttactgg ccgagaaacg gtaccaatct ttcagctctg tgcttgtcgg 1870

cttctttttg ccactggcaa aggagaattt aatgctgctt caagctcagg ggacttggct 1930

atgttaaaaa gcgttaaatg ctttcgacag tgtatttata cttacggctg cctgttaatt 1990

ttcaaaatgt tttcattgtt gctcgtgtat ccagaaaata tctcacgttg gccataaccc 2050

tggttttgtc tttttgtttc tagatgagcc cataaggtat agccatagta gagggaaacg 2110

gtagctgttt ttataaacga ctactgaact atgtgagaag acacagtgat attgtgaagt 2170

caaatggaag ttgtttcatt ttgcctaaac aaatacattt tgtgatgctg attttgcatc 2230

cagtttcagg agaagtaaaa tcagggacag gtagtcatgg agaagaggta ctactaagcc 2290

ctgcccctgt gcgtgaggca ctgagtatcc agacatccat atgaatgccc attacattct 2350

ggcttacata aacacggcta cttctaaagt atgacaaatg aaatcacagt acagtgtgtg 2410

tgtgtgtgtg tgtgtgtgtg tgtgtgtgtg tgtgtgtgta gaaagtagac gatatataaa 2470

tgtatgtccc aaaaggtata caacctccat ctatatcagc atctctgctt cttttcaaac 2530

acttttggaa gccccttcca ccttaactta ggaaaacaaa ttgtccccaa acaccaaaat 2590

ctcaaaaact catgccatgt acagacaggg gcacaccctc cgcttttgta gcactgagct 2650

gcagtgtgac tggccaggaa tgatgaggcc ccacagaagc agtgctgctc agcccccact 2710

gagaatgggg cattagctga ctggacactc atttgcatac ccggagctta cattctggct 2770

cccactatca catcgaatct atagtctgac aagcacctaa aattcacaag actctgactc 2830

tttattgata aagcaaagaa acaggcctgg ggaaccttta ggatgttact tactcctttt 2890

tatgagaagc tgagcatcat agcaaatgtt cccatgagag ggctgatgtt atttaaagca 2950

agaatttggt agcactgaag agatagctca gcagttaaga tgtaagttct gttcccagca 3010

cacatcaggt ggctcacaat ggcctgtcac tccagatgct ggggtggggg atctctcagc 3070

acctgcactt acatgcatat ccctccccat acatacataa taagcatata tatatatgta 3130

tgtgtatata tatatatgta tgtatatatg tatgtatata tgtatgtata tatatttgca 3190

ttttacaagt acctcttccc actctgagcc caatttgcta ggaaactact gggaaataag 3250

aacagggtcc ctgcttcctg cttagggtgt gtggtaaata gctgagtaat taaggaccca 3310

gggcaagcag acatgtctat gagcccaagg ttagcccaag tatcccttga cagtacataa 3370

ctcagctgct cccaaagaca gaccctctga acataaccca tctgtcagag ggagggccta 3430

ggaaactcta gtcctctgaa cttccaagga ttctttctgt caaacctcag ccagggcttt 3490

gaaatacaca ccaatctgct tatttcttcc ctttgaattt gcttccctga gaacagtcag 3550

taaatctaag aataaaatag gcaaagtaag atattgatct gataaaaaaa aaaggctaaa 3610

taaataaata aaactactat ctcaagtcag gagcagattc caaatcacct tgttactact 3670

gctggattaa aaagtaacaa caacaacaac aaaagcactc actgttctga ttttttaaaa 3730

aaatcgcctg cccaggaaac tggtactgag gggcttccag ctccccaccc cgattttttc 3790

tgaagtttct acagctctga gcgaccagtt cagtgttgat tgtgaaggga acagatgcct 3850

gcgtgcatgg ggaagctgtg tgttttcaca aagcttccca ggctgctttt gttcctccgt 3910

cactgaattc tgcagaacaa gttcaaccat tatccaagga tgtgtttgtt agggtatggg 3970

gatggagcac acagctttct gtgtgtgcca tgcgacagac actgtgggac gttgctcttt 4030

gagaatctag aaagccgact agacgctccc tgtacctccc acgggctgaa caagacatag 4090

ctttgaaagc ccaccagcaa aggtagtgag agaaggcagg ttgggaagga ggagggtgga 4150

ggaggaaaga ttttggggga ggaggggagg aacattaggg tggaggagag agatttgggg 4210

agggatgctg aggagaagta gcatcatgga tctcctttcg cattgcactg tctcatctcc 4270

tgcttataca gacttccctt agcttcctgc atggccttgc tcagcctcaa tcaccctgaa 4330

ctggtcactc cattgaaagg agactttagt ttcaaagaaa aaggaatgga ataaaagaaa 4390

gaagaagaga aaaaggaagg gaagaaaccc agggtctgag tggaagaaag ggatggggaa 4450

gactgaactg gagcttgctc tgtagtttca gccagggtaa aatcctgact gtaagattga 4510

gattgaataa caaatgggag accatttgaa tgagtggcag caaataggga acaaaatgag 4570

aactccgcaa taataagcag atcacaaagc tctctaacta tagaaagaga caggtcaggg 4630

ggctggagag atggctcagc ggttaagagc attgactgct cttccggagg tcctgagttc 4690

aaatcccagc aaccacatgg tggctcacaa ccatctgtaa caagacctga ttccctcttc 4750

tggtatctga agagacaggt tggatgtgga ggacagactt ggaagagaga cagagtctga 4810

atagaaatgg acagttgtga ctatgccaca caagcaggtt ctagagacag aaccagggac 4870

aagaagtggg aagggaacta gcttcttttg ctccaggttc tgtgctgagc cctcacttcc 4930

catgtgggtc tctcttcttc tgacaaccca gagaggtcaa aacaatcagt ctcttcacgg 4990

aacaaaggag aaagctgcct atctcccaaa catgtgatac aggctccgtt ttcctctcaa 5050

ggtacaatgc tatctattgg tcccattttc agattgataa agaaacagac aagatggcta 5110

cagagatggc tcagttttca cagtgcctgc ttctcaagag ttcagatctc tggtacccat 5170

atgagcactg ggtggatatg atgaacctaa cagtaatacc agtcttggag tgtggatact 5230

aaagatttct ggagcaacaa ggttagtaag actggacaga ttggtgggct ccgaaattga 5290

ctgagagccc ctatctcagt gaatatggag gaggagttaa caaggatgat actcaacatg 5350

aacttttggg cctccacatg tgaactcata tgcccacata caggtaccga catgggtatg 5410

cagacataca tgcatatatg acacatagag agggacgtga aaattgaaaa aaaaaagttt 5470

gacaggcaag aaaatagtaa tttatcacaa cccatgcata atgtgactct atccactgaa 5530

agaacagaag ccgtgtattt aatttaaaaa tttcagtcaa gttaaaaatg aaaaaaaaaa     5590

acactcagtg ataacaattt ttataatgct acctaagttt gtacacatac tgctggattt     5650

cttaggaaag cgaaatacag agtcatcata aaaattattg gtgagctaat ttttcttctc     5710

ctacccccat actaaatctt caactgtccc tttcccaaac atatgcacca actaccccaa     5770

ggaactgccc aagtcgcctg tgtttgcaaa actctcaagc actcatgaac aaggattgga     5830

tttcaatggt ttcgactgtt ttgctagcct ctccctagaa accctctgta aaagtgatgg     5890

ttaaaatagt aagtctacgt tattctgaaa gtacaaaact gtcttttgcc tgtctcatca     5950

taaattgtgt ctgtctgcat cgcacaaatc acaccaactg cctggcagag ctgttgacat     6010

agccctcccc ccccccccac cttaaatgga ggtgagaaaa tggctgccat taggtctgtg     6070

tttcaaaaga taatcttagg aaatctagga aaatggaatg ctgggcctac aggcgccacc     6130

aatggcagtc tgaggcatgt gcccacgggc attgtgagaa tagaggtagc ctttctagaa     6190

caggaagcca accagaccct ttgtgcaaaa tttatcattt tacaatgaca ggcaattgct     6250

tgttgttata tttgaaaaaa aaattgtcct tttctacgat acactgttga ggataaatac     6310

catactatgt ccagagttaa ctctctattc tggctgtttg aaattgtttc agctccactc     6370

tcagcaaccc tttagctaac acaatgcttt aaagtcttat tcatggctta atggagtgca     6430

ttaaagattt gactaaattt atgtttggct gcaaaaaaaa aaaaaaaaaa               6480

<210>  10
<211>  141
<212>  PRT
<213>  Mus musculus

<400>  10

Met Trp Val Leu Gly Ile Ala Ala Thr Phe Cys Gly Leu Phe Trp Leu
1               5                   10                  15

Pro Gly Leu Ala Leu Gln Ile Gln Cys Tyr Gln Cys Glu Glu Phe Gln
            20                  25                  30

Leu Asn Asn Asp Cys Ser Ser Pro Glu Phe Ile Val Asn Cys Thr Val
        35                  40                  45

Asn Val Gln Asp Met Cys Gln Lys Glu Val Met Glu Gln Ser Ala Gly
    50                  55                  60

Ile Met Tyr Arg Lys Ser Cys Ala Ser Ser Ala Ala Cys Leu Ile Ala
65                  70                  75                  80

Ser Ala Gly Tyr Gln Ser Phe Cys Ser Pro Gly Lys Leu Asn Ser Val
                85                  90                  95

Cys Ile Ser Cys Cys Asn Thr Pro Leu Cys Asn Gly Pro Arg Pro Lys
            100                 105                 110

Lys Arg Gly Ser Ser Ala Ser Ala Ile Arg Pro Gly Leu Leu Thr Thr
        115                 120                 125

Leu Leu Phe Phe His Leu Ala Leu Cys Leu Ala His Cys
    130                 135                 140

<210>  11
<211>  6159
<212>  DNA
<213>  Mus musculus

<220>
<221>  CDS
<222>  (160)..(429)
<223>  Mus musculus Ly6/Plaur domain containing 1 (Lypd1), transcript
       variant 2, mRNA

<400>  11
agtcagaccc atttgctttt cagtgagagc acagatccgg gtgtccactc cccagggctg      60

gcgctgcaaa ttcagtgcta ccagtgtgaa gaattccagc tgaacaacga ttgctcatcc     120

cctgagttca tcgtaaattg caccgtgaac gttcaagac atg tgt cag aaa gaa        174
                                           Met Cys Gln Lys Glu
                                           1               5

gtg atg gag caa agt gct ggg atc atg tac cgg aag tcg tgt gca tcg       222
Val Met Glu Gln Ser Ala Gly Ile Met Tyr Arg Lys Ser Cys Ala Ser
                10                  15                  20

tca gca gcc tgt ctc att gct tca gct ggg tac cag tcc ttc tgt tcc       270
Ser Ala Ala Cys Leu Ile Ala Ser Ala Gly Tyr Gln Ser Phe Cys Ser
            25                  30                  35

cct ggg aaa ctg aac tcc gtg tgc atc agc tgc tgc aac acc cct ctt       318
Pro Gly Lys Leu Asn Ser Val Cys Ile Ser Cys Cys Asn Thr Pro Leu
        40                  45                  50

tgc aat ggg ccg agg ccc aag aag aga ggc agc tct gcc tcg gcc atc       366
Cys Asn Gly Pro Arg Pro Lys Lys Arg Gly Ser Ser Ala Ser Ala Ile
    55                  60                  65

agg cca ggg ctt ctc acc act ctc ctg ttc ttc cac tta gcc ctc tgc       414
Arg Pro Gly Leu Leu Thr Thr Leu Leu Phe Phe His Leu Ala Leu Cys
70                  75                  80                  85

ttg gca cac tgc tga agctaaagga gatgccaacc cctgctgcct cacctgtctg       469
Leu Ala His Cys

gcccttcgtc tctcaccttc ccgagtctct tctgggtgtc cttttattct gggtagacaa     529

gggagtcttt ttgttccctc ctttcaagta acgcaagatt gccgtgcaca aatacttttg     589

taagctctga accaattcat tctgaatttc tgtgtgtagt tgaagaaaaa agcatggagc 649

agaaagtcca gaccctccca tcccaatctg gttaaccacc gccaaggcta gcctggaaga 709

accagccctt agaagtcatt gagatacgca tctgcctttc ccaaagcctt gagcttccat 769

tctgtcccag taggagtcac agtctattca gagactgctg ctgcgtgaag gtaactttgc 829

ttttgcggga ggggagagcc agtttcggct caaggcttct gaacttgcca ttcatacttc 889

ctgctcctgt aaactatttt ctggggtgga cccagctggt ttggtctctg agccagtctg 949

tggtgactca ggactcaagg gctggggctt agcctctcca ggcttggcct cagtctgaaa 1009

agtgcttaag aaaaccttgt tagttctcct ggaggaagag ttactgcgcc gggaggctag 1069

gaagatgagg gggctgcggg ctgagctggt gctgtccttg gtggagatga agcgggcacg 1129

ctggcgtttc tcttggttgg catgctgcag agtcaggcgg cagcagagca cctgccagaa 1189

caccttccgg aactgctgag aggacacgtt gtagaggaga gggttgacca cagagctgag 1249

gtagaagaag gtatcagaga agggcaggag gatcatgtat gccctgaagt acgttctggt 1309

ccagtcatgt ttgggttttg ctgcagccat gatccgtcgg atctgattgg gcatccaaca 1369

cacggccaac gtcaccacaa tcagtcctgg caggcaagaa caggagagaa aaggagacgg 1429

ggagagaaac agcatgagaa caaaaataaa taaataaaaa cccataaaat attaagcccc 1489

ttggttctgt tgcttactgg ccgagaaacg gtaccaatct ttcagctctg tgcttgtcgg 1549

cttctttttg ccactggcaa aggagaattt aatgctgctt caagctcagg ggacttggct 1609

atgttaaaaa gcgttaaatg ctttcgacag tgtatttata cttacggctg cctgttaatt 1669

ttcaaaatgt tttcattgtt gctcgtgtat ccagaaaata tctcacgttg gccataaccc 1729

tggttttgtc tttttgtttc tagatgagcc cataaggtat agccatagta gagggaaacg 1789

gtagctgttt ttataaacga ctactgaact atgtgagaag acacagtgat attgtgaagt 1849

caaatggaag ttgtttcatt ttgcctaaac aaatacattt tgtgatgctg attttgcatc 1909

cagtttcagg agaagtaaaa tcagggacag gtagtcatgg agaagaggta ctactaagcc 1969

ctgcccctgt gcgtgaggca ctgagtatcc agacatccat atgaatgccc attacattct 2029

ggcttacata aacacggcta cttctaaagt atgacaaatg aaatcacagt acagtgtgtg 2089

tgtgtgtgtg tgtgtgtgtg tgtgtgtgtg tgtgtgtgta gaaagtagac gatatataaa 2149

tgtatgtccc aaaaggtata caacctccat ctatatcagc atctctgctt cttttcaaac 2209

acttttggaa gccccttcca ccttaactta ggaaaacaaa ttgtccccaa acaccaaaat 2269

ctcaaaaact catgccatgt acagacaggg gcacaccctc cgcttttgta gcactgagct 2329

gcagtgtgac tggccaggaa tgatgaggcc ccacagaagc agtgctgctc agcccccact 2389

gagaatgggg cattagctga ctggacactc atttgcatac ccggagctta cattctggct 2449

cccactatca catcgaatct atagtctgac aagcacctaa aattcacaag actctgactc 2509

tttattgata aagcaaagaa acaggcctgg ggaaccttta ggatgttact tactcctttt 2569

tatgagaagc tgagcatcat agcaaatgtt cccatgagag ggctgatgtt atttaaagca 2629

agaatttggt agcactgaag agatagctca gcagttaaga tgtaagttct gttcccagca 2689

cacatcaggt ggctcacaat ggcctgtcac tccagatgct ggggtggggg atctctcagc 2749

acctgcactt acatgcatat ccctccccat acatacataa taagcatata tatatatgta 2809

tgtgtatata tatatatgta tgtatatatg tatgtatata tgtatgtata tatatttgca 2869

ttttacaagt acctcttccc actctgagcc caatttgcta ggaaactact gggaaataag 2929

aacagggtcc ctgcttcctg cttagggtgt gtggtaaata gctgagtaat taaggaccca 2989

gggcaagcag acatgtctat gagcccaagg ttagcccaag tatcccttga cagtacataa 3049

ctcagctgct cccaaagaca gaccctctga acataaccca tctgtcagag ggagggccta 3109

ggaaactcta gtcctctgaa cttccaagga ttctttctgt caaacctcag ccagggcttt 3169

gaaatacaca ccaatctgct tatttcttcc ctttgaattt gcttccctga gaacagtcag 3229

taaatctaag aataaaatag gcaaagtaag atattgatct gataaaaaaa aaaggctaaa 3289

taaataaata aaactactat ctcaagtcag gagcagattc caaatcacct tgttactact 3349

gctggattaa aaagtaacaa caacaacaac aaaagcactc actgttctga ttttttaaaa 3409

aaatcgcctg cccaggaaac tggtactgag gggcttccag ctccccaccc cgattttttc 3469

tgaagtttct acagctctga gcgaccagtt cagtgttgat tgtgaaggga acagatgcct 3529

gcgtgcatgg ggaagctgtg tgttttcaca aagcttccca ggctgctttt gttcctccgt 3589

cactgaattc tgcagaacaa gttcaaccat tatccaagga tgtgtttgtt agggtatggg 3649

gatggagcac acagctttct gtgtgtgcca tgcgacagac actgtgggac gttgctcttt 3709

gagaatctag aaagccgact agacgctccc tgtacctccc acgggctgaa caagacatag 3769

ctttgaaagc ccaccagcaa aggtagtgag agaaggcagg ttgggaagga ggagggtgga 3829

ggaggaaaga ttttggggga ggaggggagg aacattaggg tggaggagag agatttgggg 3889

agggatgctg aggagaagta gcatcatgga tctcctttcg cattgcactg tctcatctcc 3949

tgcttataca gacttccctt agcttcctgc atggccttgc tcagcctcaa tcaccctgaa 4009

ctggtcactc cattgaaagg agactttagt ttcaaagaaa aaggaatgga ataaaagaaa 4069

gaagaagaga aaaaggaagg gaagaaaccc agggtctgag tggaagaaag ggatggggaa 4129

gactgaactg gagcttgctc tgtagtttca gccagggtaa aatcctgact gtaagattga 4189

gattgaataa caaatgggag accatttgaa tgagtggcag caaataggga acaaaatgag 4249

aactccgcaa taataagcag atcacaaagc tctctaacta tagaaagaga caggtcaggg 4309

ggctggagag atggctcagc ggttaagagc attgactgct cttccggagg tcctgagttc 4369

aaatcccagc aaccacatgg tggctcacaa ccatctgtaa caagacctga ttccctcttc 4429

tggtatctga agagacaggt tggatgtgga ggacagactt ggaagagaga cagagtctga 4489

atagaaatgg acagttgtga ctatgccaca caagcaggtt ctagagacag aaccagggac 4549

aagaagtggg aagggaacta gcttcttttg ctccaggttc tgtgctgagc cctcacttcc 4609

catgtgggtc tctcttcttc tgacaaccca gagaggtcaa aacaatcagt ctcttcacgg 4669

aacaaaggag aaagctgcct atctcccaaa catgtgatac aggctccgtt ttcctctcaa 4729

ggtacaatgc tatctattgg tcccattttc agattgataa agaaacagac aagatggcta 4789

cagagatggc tcagttttca cagtgcctgc ttctcaagag ttcagatctc tggtacccat 4849

atgagcactg ggtggatatg atgaacctaa cagtaatacc agtcttggag tgtggatact 4909

aaagatttct ggagcaacaa ggttagtaag actggacaga ttggtgggct ccgaaattga 4969

ctgagagccc ctatctcagt gaatatggag gaggagttaa caaggatgat actcaacatg 5029

aacttttggg cctccacatg tgaactcata tgcccacata caggtaccga catgggtatg 5089

cagacataca tgcatatatg acacatagag agggacgtga aaattgaaaa aaaaaagttt 5149

gacaggcaag aaaatagtaa tttatcacaa cccatgcata atgtgactct atccactgaa 5209

agaacagaag ccgtgtattt aatttaaaaa tttcagtcaa gttaaaaatg aaaaaaaaaa 5269

acactcagtg ataacaattt ttataatgct acctaagttt gtacacatac tgctggattt 5329

cttaggaaag cgaaatacag agtcatcata aaaattattg gtgagctaat ttttcttctc 5389

ctacccccat actaaatctt caactgtccc tttcccaaac atatgcacca actaccccaa 5449

ggaactgccc aagtcgcctg tgtttgcaaa actctcaagc actcatgaac aaggattgga 5509

tttcaatggt ttcgactgtt ttgctagcct ctccctagaa accctctgta aaagtgatgg 5569

ttaaaatagt aagtctacgt tattctgaaa gtacaaaact gtcttttgcc tgtctcatca 5629

taaattgtgt ctgtctgcat cgcacaaatc acaccaactg cctggcagag ctgttgacat 5689

agccctcccc ccccccccac cttaaatgga ggtgagaaaa tggctgccat taggtctgtg 5749

tttcaaaaga taatcttagg aaatctagga aaatggaatg ctgggcctac aggcgccacc 5809

aatggcagtc tgaggcatgt gcccacgggc attgtgagaa tagaggtagc ctttctagaa 5869

caggaagcca accagaccct ttgtgcaaaa tttatcattt tacaatgaca ggcaattgct 5929

tgttgttata tttgaaaaaa aaattgtcct tttctacgat acactgttga ggataaatac 5989

catactatgt ccagagttaa ctctctattc tggctgtttg aaattgtttc agctccactc 6049

tcagcaaccc tttagctaac acaatgcttt aaagtcttat tcatggctta atggagtgca 6109

ttaaagattt gactaaattt atgtttggct gcaaaaaaaa aaaaaaaaaa 6159

<210> 12
<211> 89

<212>  PRT
<213>  Mus musculus

<400>  12

Met Cys Gln Lys Glu Val Met Glu Gln Ser Ala Gly Ile Met Tyr Arg
1               5                   10                  15

Lys Ser Cys Ala Ser Ser Ala Ala Cys Leu Ile Ala Ser Ala Gly Tyr
            20                  25                  30

Gln Ser Phe Cys Ser Pro Gly Lys Leu Asn Ser Val Cys Ile Ser Cys
        35                  40                  45

Cys Asn Thr Pro Leu Cys Asn Gly Pro Arg Pro Lys Lys Arg Gly Ser
    50                  55                  60

Ser Ala Ser Ala Ile Arg Pro Gly Leu Leu Thr Thr Leu Leu Phe Phe
65                  70                  75                  80

His Leu Ala Leu Cys Leu Ala His Cys
                85

<210>  13
<211>  6189
<212>  DNA
<213>  Mus musculus

<220>
<221>  CDS
<222>  (190)..(459)
<223>  Mus musculus Ly6/Plaur domain containing 1 (Lypd1), transcript
       variant 3, mRNA

<400>  13
ttcctgcgat cggtagggtg ttaggaaggg gcatgcggcc caaagagaag ctcccagatc      60

agaggagagc aaccaggaaa atatgggctg gcgctgcaaa ttcagtgcta ccagtgtgaa     120

gaattccagc tgaacaacga ttgctcatcc cctgagttca tcgtaaattg caccgtgaac     180

gttcaagac atg tgt cag aaa gaa gtg atg gag caa agt gct ggg atc atg     231
          Met Cys Gln Lys Glu Val Met Glu Gln Ser Ala Gly Ile Met
          1               5                   10

tac cgg aag tcg tgt gca tcg tca gca gcc tgt ctc att gct tca gct       279
Tyr Arg Lys Ser Cys Ala Ser Ser Ala Ala Cys Leu Ile Ala Ser Ala
15                  20                  25                  30

ggg tac cag tcc ttc tgt tcc cct ggg aaa ctg aac tcc gtg tgc atc       327
Gly Tyr Gln Ser Phe Cys Ser Pro Gly Lys Leu Asn Ser Val Cys Ile
                35                  40                  45

agc tgc tgc aac acc cct ctt tgc aat ggg ccg agg ccc aag aag aga       375
Ser Cys Cys Asn Thr Pro Leu Cys Asn Gly Pro Arg Pro Lys Lys Arg
            50                  55                  60

ggc agc tct gcc tcg gcc atc agg cca ggg ctt ctc acc act ctc ctg 423
Gly Ser Ser Ala Ser Ala Ile Arg Pro Gly Leu Leu Thr Thr Leu Leu
        65                  70                  75

ttc ttc cac tta gcc ctc tgc ttg gca cac tgc tga agctaaagga 469
Phe Phe His Leu Ala Leu Cys Leu Ala His Cys
    80                  85

gatgccaacc cctgctgcct cacctgtctg gcccttcgtc tctcaccttc ccgagtctct 529

tctgggtgtc cttttattct gggtagacaa gggagtcttt ttgttccctc ctttcaagta 589

acgcaagatt gccgtgcaca aatacttttg taagctctga accaattcat tctgaatttc 649

tgtgtgtagt tgaagaaaaa agcatggagc agaaagtcca gaccctccca tcccaatctg 709

gttaaccacc gccaaggcta gcctggaaga accagccctt agaagtcatt gagatacgca 769

tctgcctttc ccaaagcctt gagcttccat tctgtcccag taggagtcac agtctattca 829

gagactgctg ctgcgtgaag gtaactttgc ttttgcggga ggggagagcc agtttcggct 889

caaggcttct gaacttgcca ttcatacttc ctgctcctgt aaactatttt ctggggtgga 949

cccagctggt ttggtctctg agccagtctg tggtgactca ggactcaagg gctggggctt 1009

agcctctcca ggcttggcct cagtctgaaa agtgcttaag aaaaccttgt tagttctcct 1069

ggaggaagag ttactgcgcc gggaggctag gaagatgagg gggctgcggg ctgagctggt 1129

gctgtccttg gtggagatga agcgggcacg ctggcgtttc tcttggttgg catgctgcag 1189

agtcaggcgg cagcagagca cctgccagaa caccttccgg aactgctgag aggacacgtt 1249

gtagaggaga gggttgacca cagagctgag gtagaagaag gtatcagaga agggcaggag 1309

gatcatgtat gccctgaagt acgttctggt ccagtcatgt ttgggttttg ctgcagccat 1369

gatccgtcgg atctgattgg gcatccaaca cacggccaac gtcaccacaa tcagtcctgg 1429

caggcaagaa caggagagaa aaggagacgg ggagagaaac agcatgagaa caaaaataaa 1489

taaataaaaa cccataaaat attaagcccc ttggttctgt tgcttactgg ccgagaaacg 1549

gtaccaatct ttcagctctg tgcttgtcgg cttctttttg ccactggcaa aggagaattt 1609

aatgctgctt caagctcagg ggacttggct atgttaaaaa gcgttaaatg ctttcgacag 1669

tgtatttata cttacggctg cctgttaatt ttcaaaatgt tttcattgtt gctcgtgtat 1729

ccagaaaata tctcacgttg gccataaccc tggttttgtc tttttgtttc tagatgagcc 1789

cataaggtat agccatagta gagggaaacg gtagctgttt ttataaacga ctactgaact 1849

atgtgagaag acacagtgat attgtgaagt caaatggaag ttgtttcatt ttgcctaaac 1909

aaatacattt tgtgatgctg attttgcatc cagtttcagg agaagtaaaa tcagggacag 1969

gtagtcatgg agaagaggta ctactaagcc ctgcccctgt gcgtgaggca ctgagtatcc 2029

agacatccat atgaatgccc attacattct ggcttacata aacacggcta cttctaaagt 2089

atgacaaatg aaatcacagt acagtgtgtg tgtgtgtgtg tgtgtgtgtg tgtgtgtgtg 2149

tgtgtgtgta gaaagtagac gatatataaa tgtatgtccc aaaaggtata caacctccat 2209

ctatatcagc atctctgctt cttttcaaac acttttggaa gccccttcca ccttaactta 2269

ggaaaacaaa ttgtccccaa acaccaaaat ctcaaaaact catgccatgt acagacaggg 2329

gcacaccctc cgcttttgta gcactgagct gcagtgtgac tggccaggaa tgatgaggcc 2389

ccacagaagc agtgctgctc agcccccact gagaatgggg cattagctga ctggacactc 2449

atttgcatac ccggagctta cattctggct cccactatca catcgaatct atagtctgac 2509

aagcacctaa aattcacaag actctgactc tttattgata aagcaaagaa acaggcctgg 2569

ggaaccttta ggatgttact tactcctttt tatgagaagc tgagcatcat agcaaatgtt 2629

cccatgagag ggctgatgtt atttaaagca agaatttggt agcactgaag agatagctca 2689

gcagttaaga tgtaagttct gttcccagca cacatcaggt ggctcacaat ggcctgtcac 2749

tccagatgct ggggtggggg atctctcagc acctgcactt acatgcatat ccctccccat 2809

acatacataa taagcatata tatatatgta tgtgtatata tatatatgta tgtatatatg 2869

tatgtatata tgtatgtata tatatttgca ttttacaagt acctcttccc actctgagcc 2929

caatttgcta ggaaactact gggaaataag aacagggtcc ctgcttcctg cttagggtgt 2989

gtggtaaata gctgagtaat taaggaccca gggcaagcag acatgtctat gagcccaagg 3049

ttagcccaag tatcccttga cagtacataa ctcagctgct cccaaagaca gaccctctga 3109

acataaccca tctgtcagag ggagggccta ggaaactcta gtcctctgaa cttccaagga 3169

ttctttctgt caaacctcag ccagggcttt gaaatacaca ccaatctgct tatttcttcc 3229

ctttgaattt gcttccctga gaacagtcag taaatctaag aataaaatag gcaaagtaag 3289

atattgatct gataaaaaaa aaaggctaaa taaataaata aaactactat ctcaagtcag 3349

gagcagattc caaatcacct tgttactact gctggattaa aaagtaacaa caacaacaac 3409

aaaagcactc actgttctga ttttttaaaa aaatcgcctg cccaggaaac tggtactgag 3469

gggcttccag ctccccaccc cgattttttc tgaagtttct acagctctga gcgaccagtt 3529

cagtgttgat tgtgaaggga acagatgcct gcgtgcatgg ggaagctgtg tgttttcaca 3589

aagcttccca ggctgctttt gttcctccgt cactgaattc tgcagaacaa gttcaaccat 3649

tatccaagga tgtgtttgtt agggtatggg gatggagcac acagctttct gtgtgtgcca 3709

tgcgacagac actgtgggac gttgctcttt gagaatctag aaagccgact agacgctccc 3769

tgtacctccc acgggctgaa caagacatag ctttgaaagc ccaccagcaa aggtagtgag 3829

agaaggcagg ttgggaagga ggagggtgga ggaggaaaga ttttggggga ggaggggagg 3889

aacattaggg tggaggagag agatttgggg agggatgctg aggagaagta gcatcatgga 3949

tctcctttcg cattgcactg tctcatctcc tgcttataca gacttccctt agcttcctgc 4009

atggccttgc tcagcctcaa tcaccctgaa ctggtcactc cattgaaagg agactttagt 4069

ttcaaagaaa aaggaatgga ataaaagaaa gaagaagaga aaaaggaagg gaagaaaccc 4129

agggtctgag tggaagaaag ggatggggaa gactgaactg gagcttgctc tgtagtttca 4189

gccagggtaa aatcctgact gtaagattga gattgaataa caaatgggag accatttgaa 4249

tgagtggcag caaataggga acaaaatgag aactccgcaa taataagcag atcacaaagc 4309

tctctaacta tagaaagaga caggtcaggg ggctggagag atggctcagc ggttaagagc 4369

attgactgct cttccggagg tcctgagttc aaatcccagc aaccacatgg tggctcacaa 4429

ccatctgtaa caagacctga ttccctcttc tggtatctga agagacaggt tggatgtgga 4489

ggacagactt ggaagagaga cagagtctga atagaaatgg acagttgtga ctatgccaca 4549

caagcaggtt ctagagacag aaccagggac aagaagtggg aagggaacta gcttcttttg 4609

ctccaggttc tgtgctgagc cctcacttcc catgtgggtc tctcttcttc tgacaaccca 4669

gagaggtcaa aacaatcagt ctcttcacgg aacaaaggag aaagctgcct atctcccaaa 4729

catgtgatac aggctccgtt ttcctctcaa ggtacaatgc tatctattgg tcccattttc 4789

agattgataa agaaacagac aagatggcta cagagatggc tcagttttca cagtgcctgc 4849

ttctcaagag ttcagatctc tggtacccat atgagcactg ggtggatatg atgaacctaa 4909

cagtaatacc agtcttggag tgtggatact aaagatttct ggagcaacaa ggttagtaag 4969

actggacaga ttggtgggct ccgaaattga ctgagagccc ctatctcagt gaatatggag 5029

gaggagttaa caaggatgat actcaacatg aacttttggg cctccacatg tgaactcata 5089

tgcccacata caggtaccga catgggtatg cagacataca tgcatatatg acacatagag 5149

agggacgtga aaattgaaaa aaaaaagttt gacaggcaag aaaatagtaa tttatcacaa 5209

cccatgcata atgtgactct atccactgaa agaacagaag ccgtgtattt aatttaaaaa 5269

tttcagtcaa gttaaaaatg aaaaaaaaaa acactcagtg ataacaattt ttataatgct 5329

acctaagttt gtacacatac tgctggattt cttaggaaag cgaaatacag agtcatcata 5389

aaaattattg gtgagctaat ttttcttctc ctacccccat actaaatctt caactgtccc 5449

tttcccaaac atatgcacca actaccccaa ggaactgccc aagtcgcctg tgtttgcaaa 5509

actctcaagc actcatgaac aaggattgga tttcaatggt ttcgactgtt ttgctagcct 5569

ctccctagaa accctctgta aaagtgatgg ttaaaatagt aagtctacgt tattctgaaa 5629

gtacaaaact gtcttttgcc tgtctcatca taaattgtgt ctgtctgcat cgcacaaatc 5689

acaccaactg cctggcagag ctgttgacat agccctcccc ccccccccac cttaaatgga 5749

ggtgagaaaa tggctgccat taggtctgtg tttcaaaaga taatcttagg aaatctagga 5809

aaatggaatg ctgggcctac aggcgccacc aatggcagtc tgaggcatgt gcccacgggc 5869

attgtgagaa tagaggtagc ctttctagaa caggaagcca accagaccct ttgtgcaaaa 5929

tttatcattt tacaatgaca ggcaattgct tgttgttata tttgaaaaaa aaattgtcct 5989

tttctacgat acactgttga ggataaatac catactatgt ccagagttaa ctctctattc 6049

tggctgtttg aaattgtttc agctccactc tcagcaaccc tttagctaac acaatgcttt 6109

aaagtcttat tcatggctta atggagtgca ttaaagattt gactaaattt atgtttggct 6169

gcaaaaaaaa aaaaaaaaaa 6189

<210> 14
<211> 89
<212> PRT
<213> Mus musculus

<400> 14

Met Cys Gln Lys Glu Val Met Glu Gln Ser Ala Gly Ile Met Tyr Arg
1               5                   10                  15

Lys Ser Cys Ala Ser Ser Ala Ala Cys Leu Ile Ala Ser Ala Gly Tyr
            20                  25                  30

Gln Ser Phe Cys Ser Pro Gly Lys Leu Asn Ser Val Cys Ile Ser Cys
        35                  40                  45

Cys Asn Thr Pro Leu Cys Asn Gly Pro Arg Pro Lys Lys Arg Gly Ser
    50                  55                  60

Ser Ala Ser Ala Ile Arg Pro Gly Leu Leu Thr Thr Leu Leu Phe Phe
65                  70                  75                  80

His Leu Ala Leu Cys Leu Ala His Cys
                85

<210> 15
<211> 19
<212> RNA
<213> Artificial Sequence

<220>
<223> Oligonucleotide

<400> 15
ggcuuugcgc ugcaaaucc 19

<210> 16
<211> 19
<212> RNA
<213> Artificial Sequence

<220>
<223> Oligonucleotide

<400>  16
ggauuugcag cgcaaagcc                                                    19

<210>  17
<211>  21
<212>  DNA
<213>  Artificial Sequence

<220>
<223>  Oligonucleotide

<400>  17
ggcuuugcgc ugcaaaucct t                                                 21

<210>  18
<211>  21
<212>  DNA
<213>  Artificial Sequence

<220>
<223>  Oligonucleotide

<400>  18
ggauuugcag cgcaaagcct g                                                 21